# EUROPEAN PATENT APPLICATION

(11) **EP 4 289 461 A2**
(43) Date of publication of application: **13.12.2023**
(21) Application number: 23191157.9
(22) Date of filing: 19.12.2012
(51) Int. Cl.: A61M 16/08

(54) **RESPIRATORY TREATMENT SYSTEM INCLUDING PHYSIOLOGICAL SENSORS**

(30) Priority: 19.12.2011 US 201161577343 P
(62) Divisional of application: 12860372.7
(71) Applicant: ResMed Pty Ltd, Bella Vista, NSW 2153 (AU)
(72) Inventor: The designation of the inventor has not yet been filed
(74) Representative: Vossius & Partner Patentanwälte Rechtsanwälte mbB

(57) **Abstract**

The present invention discloses an apparatus for assessing a condition of a patient comprising a patient interface, the interface for communicating a treatment generated by a respiratory treatment apparatus to the respiratory system of a patient; and a sensing module, the sensing module adapted for removable coupling with the patient interface, the sensing module including a collector to accumulate exhaled breath that is exhaled to the patient interface, wherein the sensing module further comprises an electrochemical sensor, the electrochemical sensor configured to sense a chemical accumulated by the collector and to generate a signal indicative of the chemical in the collector.

## Description

### CROSS REFERENCE TO RELATED APPLICATIONS

This application claims the benefit of the filing date of United States Provisional Patent Application No. 61/577,343 filed December 19, 2011, the disclosure of which is hereby incorporated herein by reference.

### FIELD OF THE TECHNOLOGY

The present technology relates to ' physiological detectors such as chemical sensors, biochemical sensors or sample collectors that may be suitable for detecting conditions of a patient such as from breath, saliva or perspiration. Example embodiments may be incorporated with components of respiratory treatment apparatus, such as a pressure treatment mask or other patient interface.

### BACKGROUND OF THE TECHNOLOGY

Different forms of respiratory treatment exist for the different respiratory related conditions. One form of respiratory treatment therapy, typically for patients with obstructive sleep apnea (OSA), is continuous positive airway pressure (CPAP) applied by a blower (compressor) via a connecting hose and mask. The positive pressure may be used to prevent collapse of the patient's airway during inspiration, thus preventing recurrent apnoeas or hypopnoeas and their sequelae. Such a respiratory treatment apparatus can function to generate a supply of clean breathable gas (usually air, with or without supplemental oxygen) at the therapeutic pressure or pressures that may change to treat different events but may remain approximately constant across a given cycle of the patient respiration cycle (i.e., inspiration and expiration) or may be reduced for comfort during each expiration (e.g., bi level CPAP).

Mechanical ventilators may also provide respiratory treatment. Mechanical ventilators may be used to treat patients who are incapable of spontaneous respiration due to trauma, pathology or both.

Respiratory treatment apparatuses may typically include a flow generator, an air filter, a mask or cannula, an air delivery conduit connecting the flow generator to the mask, various sensors and a microprocessor-based controller. The flow generator may include a servo-controlled motor and an impeller. The flow generator may also include a valve capable of discharging air to the atmosphere as a means for altering the pressure delivered to the patient as an alternative to motor speed control. The sensors measure, amongst other things, motor speed, gas volumetric flow rate and outlet pressure, such as with a pressure transducer, flow sensor or the like. The apparatus may optionally include a humidifier and/or heater elements in the path of the air delivery circuit. The controller may include data storage capacity with or without integrated data retrieval/transfer and display functions.

Patients undergoing therapy with respiratory treatment apparatuses often suffer from other physiological conditions or diseases which require monitoring, and in some cases treatment. In the case of ventilator dependent patients, they often present with multiple co morbidities and are difficult to manage clinically. These patients are often non ambulatory, further increasing the severity of their conditions.

Sensors, (e.g. biochemical sensors) for detecting various analytes have been developed. Many disease processes create by products, which may be eliminated from the body through, among other things, expiration and perspiration. These by products, may be in the form of volatile organic compounds (VOCs) in the breath, or other types of analytes in the breath, saliva or perspiration. Electrochemical sensors have been developed to detect various analytes in the breath of a patient.

As demonstrated by the present technology, there may be a need for improvement of respiratory treatment apparatuses for monitoring patient conditions.

### BRIEF SUMMARY OF THE INVENTION

In accordance with an aspect of the present technology, a patient interface system is provided for delivery of a supply of breathable gas to the airways of a patient during sleep.

In accordance with an aspect of the present technology, an apparatus is provided for treatment of sleep disordered breathing.

In accordance with an aspect of the present technology, a patient interface system is provided that is adapted to retain a portion of an exhaled breath.

In accordance with an aspect of the present technology, a patient interface system is provided that comprises an adsorbent material.

In accordance with an aspect of the present technology, a patient interface system is provided that comprises an absorbent material.

In accordance with one form of the present technology, a mandibular advancement device (MAD), or mandibular repositioning device (MRD), or other dental orthosis comprises a sample collection structure or sample collection chamber.

In accordance with an aspect of the present technology, an apparatus for treating sleep disordered breathing is provided that is adapted to collect and/or detect the presence of compounds in the exhaled breath of a patient.

In accordance with an aspect of the present technology, an apparatus for treating sleep disordered breathing is provided that is adapted to collect and/or detect the presence of compounds in the exhaled breath of a patient indicative of cancer.

In accordance with an aspect of the present technology, an apparatus for treating sleep disordered breathing is provided that is adapted to detect whether the patient also has diabetes.

In accordance with an aspect of the present technology, an apparatus for treating sleep disordered breathing is provided that is adapted to detect whether the patient also has metabolic digestion.

In accordance with an aspect of the present technology, an apparatus for treating sleep disordered breathing is provided that is adapted to detect whether the patient also has oxidative stress.

In accordance with an aspect of the present technology, an apparatus for treating sleep disordered breathing is provided that is adapted to detect whether the patient also has asthma.

In accordance with an aspect of the present technology, an apparatus for treating sleep disordered breathing is provided that is adapted to detect whether the patient also has COPD.

In accordance with an aspect of the present technology, an apparatus for treating sleep disordered breathing is provided that is adapted to detect whether the patient also has cardiac autonomic control.

In accordance with an aspect of the present technology, an apparatus for treating sleep disordered breathing is provided that is adapted to detect whether the patient also has bronchitis.

In some embodiments of the present technology, an apparatus may be configured for assessing a condition of a patient. The apparatus may contain a patient interface, and the interface may be used for communicating a treatment generated by a respiratory treatment apparatus to the respiratory system of a patient. The apparatus may also contain a sensing module that may be adapted for removable coupling with the patient interface. The sensing module may include a collector to accumulate exhaled breath that is exhaled to the patient interface. In some embodiments, the sensing module of the technology may also include a plurality of sample collectors. The sample collectors may contain an absorbent material, and may also be adapted for replacement within a sensing module. In some embodiments of the technology, the collector may contain an absorbent material, the absorbent material may also include a preservative for preserving an analyte collected from the exhaled breath. In some embodiments, the technology may also include a sensing module that includes an electrochemical sensor configured to sense a chemical in breath condensate accumulated by a collector of the present technology. The sensing module of the present technology may also include a plurality of electrochemical sensors.

In some embodiments, the present technology may also include a respiratory treatment apparatus with a flow generator and a controller, including at least one processor. The controller controls the flow generator to generate a pressure treatment to the patient interface. In such an embodiment, the technology may also include an electrochemical sensor configured to sense a chemical accumulated by the collector and generate a signal indicative of a chemical of a collector. Furthermore, such an embodiment may include a controller that may be coupled with the sensor and the processor and be configured to store a measure derived from the signal. In yet another embodiment, the sensor of the present technology may be integrated along with a housing of the flow generator, and may also comprise an electrochemical sensor.

In some embodiments, a processor may be configured to synchronize an activation of the electrochemical sensor with an expiratory phase of a detected breath cycle. The processor may also be configured to activate the electrochemical sensor after a set period of time. Such a set period of time may comprise a number of treatment sessions with a respiratory treatment apparatus, and may also comprise a breath cycle count. The set period of time may also comprise a timed period of use of the respiratory treatment apparatus. In some such embodiments, a collector of the sensing module may include a variable aperture controlled by the processor. In such an embodiment, the processor may be configured to synchronize an opening of the variable aperture with an expiratory phase of a detected breath cycle. In yet another embodiment of the present technology, the processor may be configured to open the variable aperture for a set period of time. Such a set period of time may comprise a breath cycle count and/or a timed period of use of the respiratory treatment apparatus. A set period of time, according to some such embodiments may also include a number of treatment sessions with the respiratory treatment apparatus.

In some embodiments, the processor may also be configured to compare a measure derived from the signal and a threshold value. The processor may also be configured to generate a warning based on a comparison of a measure derived from the signal and a threshold value. Furthermore, in some embodiments, the processor may be configured to detect a quantity of an analyte from exhaled breath. Such embodiments may also contain a sensing module having one or more sensors such as a peroxide sensor, a nitrous oxide sensor, an acetone sensor, a carbon dioxide sensor, a pH sensor, a glucose sensor, and/or a lactate sensor.

In some embodiments of the present technology, an apparatus is configured for assessing a condition of a patient. The apparatus may include a patient interface configured to direct a flow of breathable gas. It may further include a flow generator adapted to couple with the patient interface. The flow generator may be further adapted to generate a flow of breathable gas to the patient interface. The apparatus may also include a controller including at least one processor. The processor may be configured to control a pressure treatment protocol with the flow generator. The apparatus may also include a sensing module containing at least one electrochemical sensor. The sensing module may be adapted for removable coupling with the patient interface and include a collector to accumulate exhaled breath condensate that is exhaled to the patient interface. The electrochemical sensor may also be configured to sense a chemical accumulated by the collector and generate a signal indicative of a chemical of the collector. In such embodiments, a controller may be coupled with a sensor and processor thereof, and may be configured to store a measure derived from the signal. Optionally, the sensing module may also include a plurality of sample collectors wherein such sample collectors may contain an absorbent material and may also be adapted for replacement within the sensing module. In such an embodiment, the absorbent material may also include a preservative for preserving an analyte collected from the exhaled breath.

Optionally, such embodiments may also include a sensing module having one or more sensors of the following sensors: a peroxide sensor, a nitrous oxide sensor, an acetone sensor, a carbon dioxide sensor, a pH sensor, glucose sensor, and/or a lactate sensor. The processor may also be configured to synchronize the activation of one or more of the sensors of the sensing module with an expiratory phase of the detected breath cycle. A processor of such embodiments may also be configured to activate one or more of the sensors of the sensing module after a set period of time. Such a set period of time may be a number of treatment sessions with the respiratory treatment apparatus, and/or may also be a breath cycle count. Such a set period of time may be a time period of use of the respiratory treatment apparatus.

In some such embodiments, a collector may also contain a sensing module with a variable aperture controlled by the processor. Such a processor may be configured to synchronize an opening of the variable aperture with an expiratory phase of a detected breath cycle. In such an embodiment, the processor may be configured to open the aperture for a set period of time. Such a set period of time may include a breath cycle count or a time period of use of the respiratory apparatus and/or a number of treatment sessions with the respiratory treatment apparatus. A processor of the present technology may also be configured to compare a measure derived from the signal and a threshold value.. The processor may also be configured to generate a warning based on a comparison of a measure derived from the signal and a threshold value. Such an apparatus may also be configured to detect a quantity of an analyte from exhaled breath.

Some examples of the present technology may include an apparatus for assessing a condition of a patient. The apparatus may include a patient interface, the interface for communicating a treatment generated by a respiratory treatment apparatus to the respiratory system of a patient. The patient interface may include a sensing module configured as a collector to accumulate saliva or mucus that is in contact with the patient interface. In some cases, the sensing module may further include an electrochemical sensor, the electrochemical sensor configured to sense a chemical in saliva or mucus accumulated by the collector. In some such cases, the patient interface may comprise a nasal cannula and the sensing module may comprise at least one prong of the nasal cannula to contact mucosa.

Some examples of the present technology may include apparatus for assessing a condition of a patient. The apparatus may include a mandibular advancement device. The mandibular advancement device may include a sensing material. The material may be configured as a collector to accumulate saliva that is in contact with the mandibular advancement device. In some cases, the mandibular advancement device further include a collection chamber.

Various aspects of the described example embodiments may be combined with aspects of certain other example embodiments to realize yet further embodiments.

Other features of the technology will be apparent from consideration of the information contained in the following detailed description, abstract, drawings and claims.

### BRIEF DESCRIPTION OF THE DRAWINGS

FIG. 1 is a block diagram of an example respiratory support system, wherein the physiological sensor(s) are added on to the system as an accessory or attachment;
FIG. 2 is a block diagram of an example respiratory support system, wherein the physiological sensor(s) are integrated;
FIG. 3 is an example respiratory treatment apparatus with physiological sensor(s) and a controller;
FIG. 3A is a left side view of an example respiratory mask with a breath collection device;
FIG. 4 is front view of a representation of a physiological sensor;
FIG. 4A is a top view of a physiological sensor contained in a sensor housing;
FIG. 4B is a top down view of a sensor housing containing two physiological sensor(s);
FIG. 4C is a top down view of a sensor housing containing three physiological sensor(s);
FIG. 4D is a top down view of a sensor housing containing four physiological sensor(s);
FIG. 5 is an example nasal respiratory treatment mask with integrated physiological sensor(s);
FIG. 6 is an example nose and mouth respiratory treatment mask with integrated physiological sensor(s);
FIG. 6A is an example mask, as seen in FIGS. 5 and 6, which contains a sensor attached to the underside of the mask frame;
FIG. 6B is an example mask, as seen in FIGS. 5 and 6, which contains two sensors attached to the underside of the mask frame;
FIG. 7 is a left side view of another respiratory treatment mask with physiological sensor(s) integrated in a headgear support for the mask;
FIG. 8 is a right side view of the respiratory treatment mask of FIG. 7 with' additional physiological sensor(s) integrated in the headgear support for the mask;
FIG. 9 is a right side view of another respiratory treatment mask with a physiological sensor integrated in a headgear support for the mask;
FIG. 10 is a left side view of the respiratory treatment mask of FIG. 9 with an additional physiological sensor integrated in the headgear support for the mask;
FIG. 11 is a front view of a respiratory treatment mask containing a physiological sensor attached to the end of a gas delivery tube;
FIG. 12 is a left side view of the respiratory treatment mask of FIG. 11;
FIG. 13 is a front view of a respiratory treatment mask containing a physiological sensor attached to the frame of the mask;
FIG. 14 is a left side view of the respiratory treatment mask of FIG. 13;
FIG. 15 is a front view of a respiratory treatment mask containing three physiological sensor(s) attached to the frame of the mask;
FIG. 16 is a left side view of the respiratory treatment mask of FIG. 15;
FIG. 17 is a front view of a respiratory treatment system, in which a physiological sensor is attached to a nasal cannula;
FIG. 18 is a cross section of a gas delivery tube containing one or more physiological sensor(s) on the inside of the tube;
FIG. 19 is a left side view of a gas delivery tube with coupling mechanisms for coupling to a physiological sensor;
FIG. 20 is a left side view of a gas delivery tube with an integral physiological sensor;
FIG. 21 is a top view of a gas delivery tube with a bypass tube and a physiological sensor;
FIG. 22 is a cross section of a coupling mechanism containing electrical contacts;
FIG. 23 is a front view of a respiratory treatment apparatus containing a flow generator and pressure sensor;
FIG. 24 is a block diagram depicting various components of a controller of the present technology;
FIG. 25 is a functional block diagram depicting various processes performed by a controller in the present technology;
FIG. 26 is a block diagram depicting a respiratory support system with a cascading sensor configuration.

### DETAILED DESCRIPTION

### INTRODUCTION (GENERAL)

The present technology involves methods and apparatuses for providing respiratory treatment for patients, monitoring and detecting various physiological characteristics of a patient, and/or treating the patient according to those physiological characteristics. A respiratory treatment apparatus can be of any type commonly known in the art. These may include, but are not limited to ventilators, respirators, continuous positive airway pressure (CPAP) apparatuses, bi level positive airway pressure device (VPAP) and automatic positive airway pressure apparatuses (APAP). Respiratory treatment apparatuses useful in the present technology generate breathable gas for a patient, and may include a flow generator such as a servo controlled blower. The blower may typically include an air inlet and impeller driven by a motor (not shown).

Respiratory treatment apparatuses are generally in fluid communication with a patient through tubing, and some type of patient interface. Patient interfaces are known to those of ordinary skill in the art and include, but are not limited to nasal masks, nose & mouth masks, full face masks, nasal pillows and nasal cannulas and the delivery conduits coupled therewith. The mask or cannula may receive airflow from the patient's respiratory system via the patient's mouth and/or the patient's nares. The respiratory treatment apparatus may include a vent to provide an intentional leak.

Air is exchanged between a patient and a respiratory treatment apparatus via a gas delivery tube or conduit. Generally, the gas delivery tube or conduit attaches to the respiratory treatment apparatus at one end, and the patient interface at the other end.

The present technology also employs coupling mechanisms which may be implemented on physiological sensor(s) and/or sample collector, as well as a gas delivery tube, conduit and/or a patient interface. By way of example only, the coupling mechanisms may include screw threads, hose clamps, locking mechanisms, bayonet mounts and press fit configurations. The coupling mechanisms may also include electronic contacts or wiring to allow for the transmission of signals from the physiological sensor(s), to the respiratory treatment device, and vice versa. Regardless of the structure employed, the coupling mechanism provides a secured connection for treatment while permitting an easily removable design for maintenance or replacement by the patient or health care provider. The present technology, because of the coupling mechanisms described herein, may also include kits to house the sensors and/or sample collectors. Thus, these kits may contain one or more physiological sensor(s) and may be adapted for coupling with, patient interfaces for different types of respiratory treatment apparatuses. Similarly, different kits may provide different collections of sensors for patients having or at risk for different conditions.

In accordance with one form of the present technology, the presence of ketones in exhaled breath of a patient being treated for sleep disordered breathing is used to detect the presence of diabetes and/or metabolic digestion in the patient.

In accordance with one form of the present technology, the presence of acetone in exhaled breath of a patient being treated for sleep disordered breathing is used to detect the presence of diabetes and/or metabolic digestion in the patient.

In accordance with one form of the present technology, the presence of glucose in exhaled breath of a patient being treated for sleep disordered breathing is used to detect the presence of diabetes in the patient.

In accordance with one form of the present technology, the presence of insulin in exhaled breath of a patient being treated for sleep disordered breathing is used to detect the presence of diabetes in the patient.

In accordance with one form of the present technology, the presence of one or more of Leukotriene B4, Interleukin 6 and H2O2 in exhaled breath of a patient being treated for sleep disordered breathing is used to detect the presence of one or more of oxidative stress, asthma, diabetes and COPD in the patient.

In accordance with one form of the present technology, the presence of hs CRP in exhaled breath of a patient being treated for sleep disordered breathing is used to detect the presence of cardiac autonomic control in the patient.

In accordance with one form of the present technology, the pH of exhaled breath of a patient being treated for sleep disordered breathing is used to detect the presence of asthma or bronchitis in the patient.

In accordance with one form of the present technology, the conductivity of exhaled breath of a patient being treated for sleep disordered breathing is used to detect the presence of asthma or bronchitis in the patient.

In accordance with one form of the present technology, a sensor contacting the skin of the patient is combined with a sensor to detect compounds in exhaled breath of the sleeping patient to detect the presence of a disease state, while treating the patient for sleep disordered breathing.

Thus, embodiments of the present technology may provide a comprehensive respiratory treatment system, which includes one or more physiological sensor(s) and/or sample collectors. The physiological sensor(s) (e.g. biochemical sensors) may be adapted to generate various physiological signals, such as signals that are representative of analytes or biomarkers in a patient that may be attributable to patient breath or perspiration such as breath or perspiration that may be collected with a sample collector associated with the sensor.

In some embodiments of the technology, one or more physiological, sensor(s) may be electrochemical sensors configured to detect biochemical analytes in the breath or perspiration of a patient. Optionally, the signal(s) may be evaluated by one or more processors, such as a processor of the respiratory treatment apparatus. For example, the processor of the respiratory treatment device may compare data or measurements derived from the signal (s) to one or more thresholds. Suitable thresholds for detection may be determined empirically such that the comparison may be indicative of different disease conditions or changes in disease conditions that relate to the detection capabilities of the sensors. Based on an analysis and control algorithm in the flow generator of the respiratory treatment apparatus, detection of VOC, chemical or other analyte levels above a certain threshold or patterns of such levels may result in a message being generated on a display, an audio alarm (if acute) recommending to seek medical help. In some cases, the information may be transmitted such as by wired or wireless communication to the patient's physician. In some embodiments, and depending on the nature of the detected condition, the respiratory treatment apparatus may generate or trigger an automatic emergency telephone call (e.g., a 911 call) and play an automated voice message such as with name, address and detected condition information to request more immediate help by phone.

Thus, embodiments of the technology may include a controller with a processor for receiving and processing the physiological signals. Such processing may include analysis of biochemical data obtained by the physiological sensors. Once the signals are received by the controller, the signals may be stored as data, in at least one memory. The controller, which may also contain at least one processor, may utilize the stored data, as well as real time data, to make various treatment recommendations or alerts. The analysis controller may be integrated into the respiratory treatment system, or may be an add on accessory or attachment for such an apparatus.

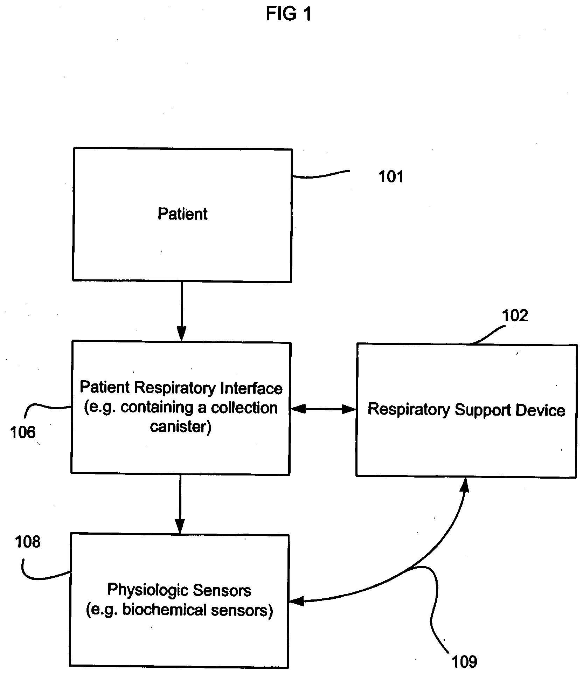

FIG. 1 is a block diagram of an example respiratory treatment system representing one embodiment of the present technology. In FIG. 1, a patient 101, is connected to a respiratory treatment apparatus 102, via a patient interface 106. The physiological sensor(s) 108, such as a sensor to detect a substance or chemical of the breath of a patient, in this embodiment may be attached to the patient interface 106 as an accessory or add on. As shown, physiological sensor(s) 108 may be in electronic communication 109 with respiratory treatment apparatus 102.

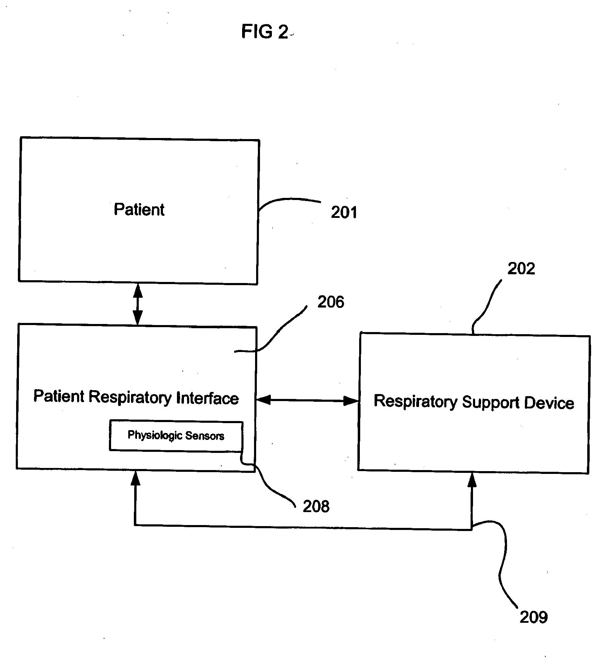

FIG. 2 is a block diagram of an example respiratory treatment system representing an alternative embodiment of the present technology. In FIG. 2, a patient 201 is connected to a respiratory treatment device 202 via a patient interface 206. In this embodiment, the physiological sensor(s) 208 are integrated within the patient interface 206. The physiological sensor(s)(e.g. biochemical sensors) 208 are in communication with the respiratory treatment apparatus 202, via electronic circuitry 209.

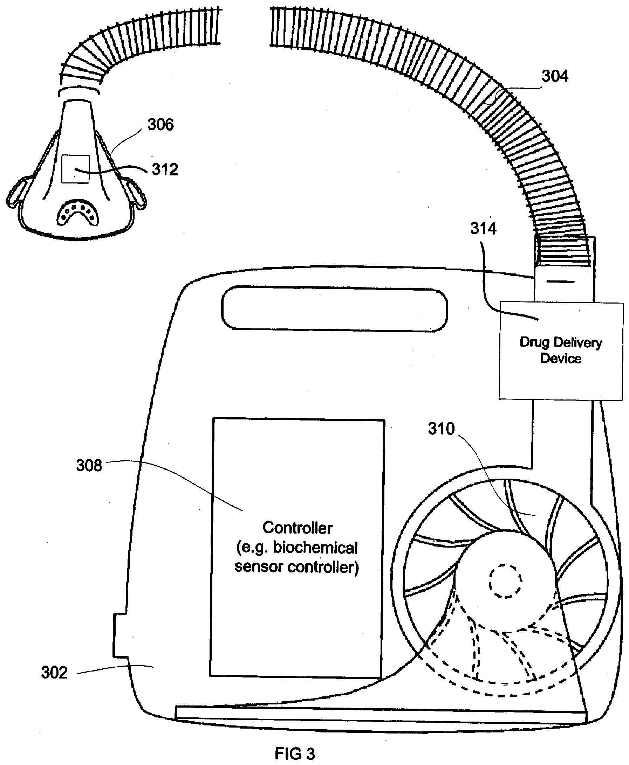

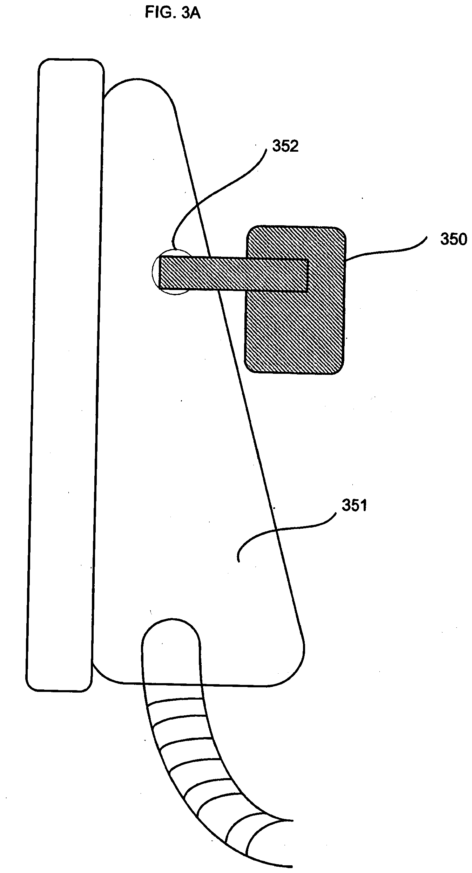

FIG. 3 depicts an example of a respiratory treatment system. Respiratory treatment apparatus 302 contains a controller 308 and a flow generator 310. Respiratory treatment apparatus 302, is connected to tube 304. Tube 304 is in fluid communication with a patient interface 306. Patient interface 306 contains at least one physiological sensor 312, which is in electronic communication with respiratory treatment apparatus 302 via electronic communication means.

Optionally, the respiratory treatment apparatus 302, or patient interface 306 may also include a drug delivery device 314 for delivering medication to a patient such as via an aerosolized delivery system. The drug delivery device may be prefilled with a medication, specific for a particular patient's needs. The respiratory treatment system may control the delivery of the medication based on specific signals. For example, the medication may be stored in an onboard chamber, within the respiratory treatment device 302. Upon receiving a signal from the controller, medication may be released from the chamber, into a conduit (not shown) contained within the patient interface 306. This conduit may be the same structure as those typically used in CPAP machines to deliver humidified air to the patient. Alternatively, the medication may be stored remotely from the respiratory device in a reservoir contained within the patient interface. In this instance, an electronic signal from the controller, that may be generated in response to an analysis of a signal from physiological sensor 312, may signal an electromechanical component on the drug delivery device 314 to release the medication. In this instance, the medication may be released into the stream of air administered to the patient during the inspiratory phase of respiration. This timing allows for the medication to aerosolize in the air stream.

### PHYSIOLOGICAL SENSORS/SAMPLE COLLECTORS

Human breath contains many analytes, which may be indicative of various physiological conditions. For example, trace amounts of volatile organic compounds may be detected in a patient's breath. The detection of such trace amounts may be implemented in diagnostic technologies. Sensors, such as small array sensors may be used to detect one or more of these volatile organic compounds. By way of representative example, such sensors may be types of nanodetectors, such as those and in particular, infrared spectroscopic detection, such as that developed by Applied Nanodetectors. In some cases, such sensors may optionally employ metal oxide or "MOx" receptors. Other types of sensors may include those employing the use of spectroscopic or light analysis, may be useful to sense analytes such as acetone. In embodiments of the present technology, spectroscopic detection may be used in conjunction with an external, light emitting source. Optionally, such embodiments may be used in conjunction with a sample collector as shown in FIG. 3A, such as a collection canister 350 that may be attachable to a respiratory patient interface 351. For example, a collection canister may be attachable (e.g., threaded in or onto) to an aperture of the mask. The aperture may be suitably located with respect to the patient's nares or mouth for the collection of expired breath or perspiration from the skin of the patient. The canister may be detachable for analysis by a separate sensor that may optionally be integrated with the respiratory treatment device. Thus, in some embodiments, the canister may be detached from a canister mount on a mask and attached to a canister mount associated with a sensor of the respiratory treatment apparatus for analysis. In such a case, the canister mount of the mask and respiratory treatment apparatus may have the same or similar attachment configuration (e.g., aperture size and/or coupling mechanism.) Alternatively, the sample collector or collection canister may also be attachable to a sensor such that both may be mounted to the patient interface via the aperture.

Optionally, any of the sensors and/or collectors described in this specification may be located in or coupled to an expiratory duct, such as an expiratory duct of a dual-limb ventilator, so as to permit, for example, capturing and/or analysis of a whole expiratory breath.

Different sensors may be employed in different embodiments. For example, detection of increased levels of nitrous oxide in the breath may be indicative of chronic obstructive pulmonary disease (COPD) or asthma. Thus, some embodiments may employ a nitrous oxide sensor. In some embodiments, detection of CO2 may be indicative of metabolic or respiratory alkalosis and acidosis. Increased CO2 levels in the breath may also indicate diabetes and renal failure. Thus, some embodiments may employ a carbon dioxide sensor. Low pH may be indicative of many disorders, including asthma and acidosis. Thus, in some embodiments, a pH sensor may also be implemented to detect pH levels in the condensate of a patient's breath. Similarly, a detection of increased or existing levels of peroxide may be appropriate for patients with COPD and asthma to detect inflammation. Thus, in some embodiments a peroxide sensor may be implemented. For example, an ECoCheck sensor from Carl Reiner GmbH (www.carlreiner.at) may be implemented. Similarly, a detection of increased or existing levels of lactate may be appropriate for various metabolic conditions. Thus, in some embodiments a lactate sensor may be implemented. In some embodiments, a chemical sensor for detecting ketone bodies may be utilized.

In yet another embodiment, at least one of the sensors may be implemented to detect and assess acetone levels in the breath. Acetone levels may be useful in detecting metabolic conditions such as diabetic ketoacidosis. Optionally, other electrochemical sensors may be used to detect cancer or gastrointestinal disorders such as H. pylori and irritable bowel syndrome, for example, by detection of one or more VOCs.

Exhaled breath condensate in COPD patients may be acidified, a condition known as acidopnea. Salivary acids and bases may also be a useful for assessing COPD and other pulmonary inflammatory diseases such as asthma. The presence of acidic volatile substances such as NH₄⁺ and acetate in saliva may be indicative of COPD, or other inflammatory conditions of the pulmonary system. The presence of nonvolatile cations such as K⁺ and Ca₂⁺ may also prove to be useful diagnostic tools. Similarly, the presence of other acids and bases in saliva could be indicative of non-pulmonary related diseases such as GERD. The technology described herein provides devices and sensors for detecting and analyzing various salivary acids and bases, as well as other analytes in saliva.

Some embodiments of the present technology may also be implemented with one or more electrical sensors for obtaining cardiac signals from a patient. These sensors may be utilized in any type of cardiac sensing, including electrocardiograms.

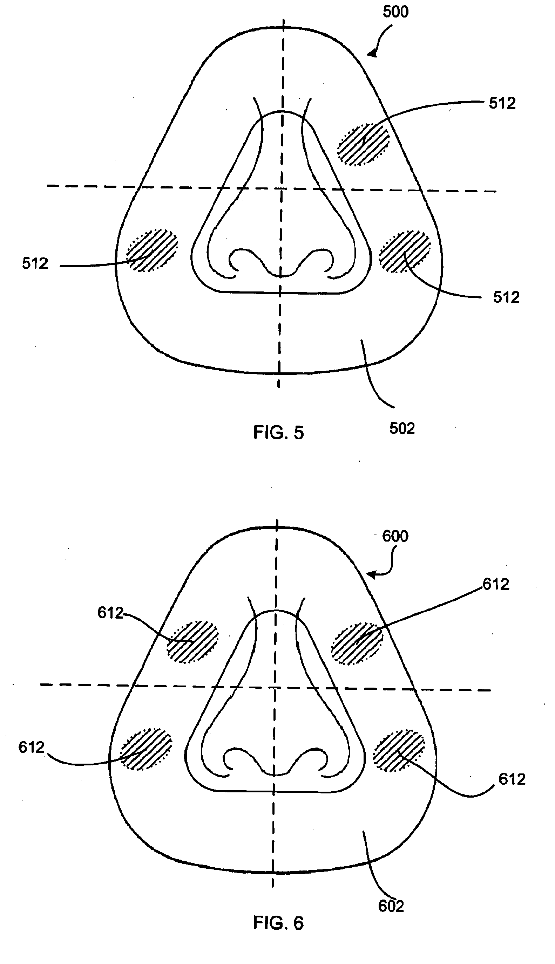

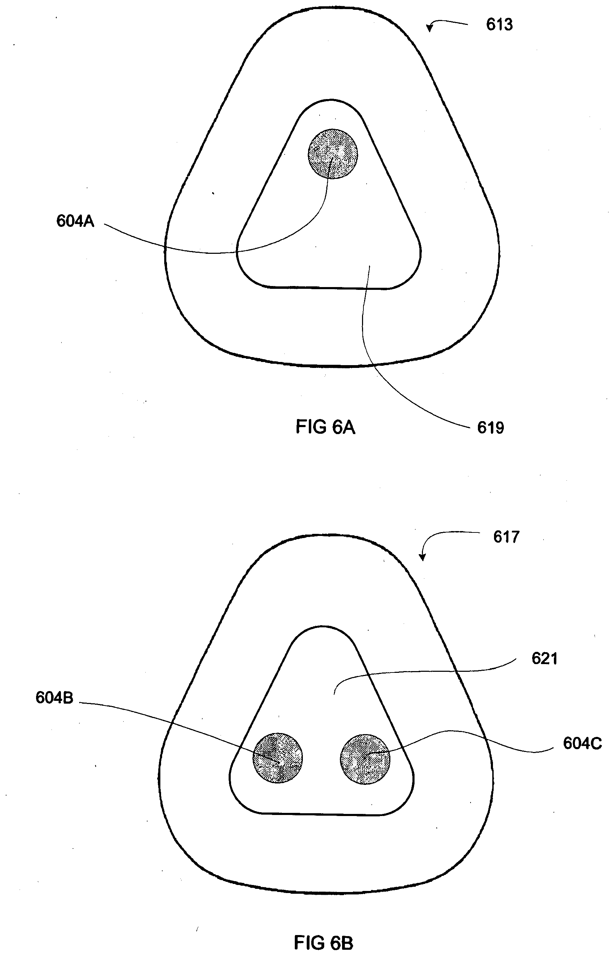

Another type of sensor that may be implemented in embodiments of the present technology are those used for monitoring blood gas such as oxygen saturation. For example, a transcutaneous pulse oximeter may be added to the rim of the mask. In one example, pulse oximetry sensors may be mounted on the underside of a face mask cushion as depicted in FIGS. 5 and 6.

By way of further example, some of the sensors of the present technology may also, or alternatively, be implemented to detect other signals. For example, facial electrodes configured or positioned as previously described herein may also be implemented to detect head, face or skin temperature by equipping one or more electrodes as a facial contact thermistor. Similarly, the electrodes discussed herein may be implemented to detect head, face or skin impedance and/or galvanic skin response (skin conductance). Such signals may then be processed by the aforementioned components to derive further metrics for patient analysis. For example, one or more of the signals previously mentioned may be processed to assess sympathetic activation of the patient.

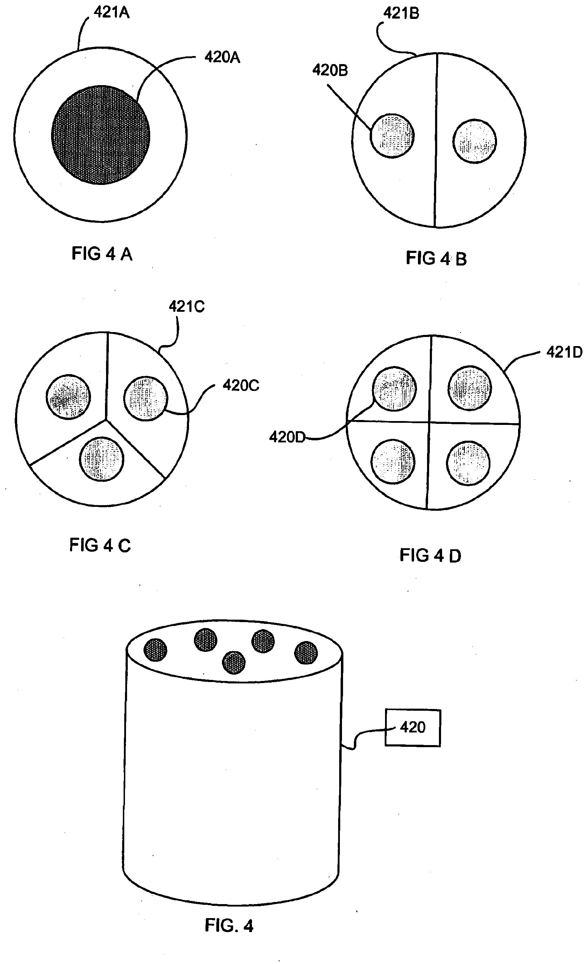

As illustrated in FIG. 4, a physiological sensor(s) 420 or a sample collector may be self contained. They may also be contained in a sensing module 421A D. Any suitable configuration of physiological sensor(s) or sample collector within a sensing module may be implemented. FIGS. 4A 4D are examples of different configurations of physiological sensor(s) or sample collector(s) contained within a sensing module that may optionally include one or more sensors and/or one or more sample collectors. FIG. 4A depicts a sensing module 421A, configured to contain one physiological sensor 420A. FIG. 4B depicts a sensing module 421B configured to contain two physiological sensor(s) 420B. FIG. 4C depicts a sensing module 421C, configured to contain three physiological sensor(s) 420C. FIG. 4D depicts a sensing module 421D, configured to contain four physiological sensor(s) 420D. It is contemplated by the present technology that the sample collector or breath collection canister may be adapted and integrated into a respiratory treatment system in the same ways as the physiological sensor(s) or sensor- module of the present technology (e.g., as shown in FIGS. 4, 4A, 4B, 4C, 4D, 5, 6, 6A, 6B and 11 23.)

The sensing module may be configured to securely hold or contain physiological sensor(s) and/or sample collectors such as one or more breath collection canisters and provide a structure for coupling with a patient interface. In some embodiments of the present technology, the physiological sensor(s) may be removable and interchangeable within the sensing module. The physiological sensor(s) may be secured within the sensing module by means including but not limited to a press fit, or threaded arrangement. This functionality allows for the exchange of different sensors within a given module, providing a user the ability to use multiple sensors with one module. Similarly, the module may also permit changing of sample collectors of the module. It also may provide the ability to use different combinations, or types of sensors and collectors, thus enabling a clinician the ability to monitor various physiological conditions simultaneously, depending on the needs of a patient. The sensing module may have a configuration to easily secure to, and easily be removed from a patient interface. This embodiment is also useful as it allows physiological sensor(s) or sample collectors to be replaced when they are no longer needed or their useful life has been exceeded.

While the sensors and housing may be cylindrical as illustrated in the figures, they may also be any other shape or size practical for use in the technology (e.g., fits within a patient interface, gas conduit, or tube).

As previously mentioned, the present technology may be implemented to detect trace analytes over an extended period of time. Conditions such as cancer and infections exist that may yield biomarkers, which may be detectable in the breath of a patient. Some of these biomarkers, or analytes may only be present in trace amounts and may be unable to be detected by some sensors known in the art. In the present technology, a collection canister or module may be configured with an absorbent substance to collect trace analytes over a period of time. Optionally, the absorbent substance may be treated with some type of growth medium, if the analyte being sampled is microbiological in nature. The absorbent material may also be treated with a chemical fixative to preserve any cellular products for later sampling or assays, including but not limited to nucleic acid amplification assays.

In the present technology, sensing may be conducted and analyzed by a processor, such as the controller of a respiratory treatment device, in real time. Certain analytes may be in significant enough quantities to be detected in real time and processed by the system. In contrast, some analytes (e.g., immunological markers and pH) may only appear in trace amounts. The present technology provides solutions to this dichotomy in detection parameters. For example, analytes detectable in trace amounts may be collected by the sensing module, if the sample collector of the mask allows an accumulation of breath gas or breath condensate. Breath condensate may include saliva and/or sputum. Over a period of time (e.g., between 12 hours and two weeks) analytes in a patient's breath may be collected for analysis. Once collection is complete, the analysis may be conducted with a sensor controlled by with the respiratory treatment system as described herein, or the sample collector may be removed and analyzed by another device. In some cases, such as in the case of a sensor having a sample collector, the sensor may have a delayed detection period controlled by a processor such that the sensor may be automatically activated by the system after a timed collection period has transpired. Optionally, the timed collection period may be associated with the amount of use of the treatment apparatus (e.g., a time of operation of the treatment apparatus). Optionally, the timed collection period may correspond to a number of breaths made by a patient into a collector. In some cases, the activation of the sensor may be coordinated with patient respiration. For example, the sensor may be triggered for sensing only during patient expiration.

Similarly, an access port to a collector or sensor may be opened only during patient expiration. For example, a sample collector or sensor may include a variable aperture, such as an electro mechanically controlled collector cover, a vent cover or shutter. The variable aperture may be controlled by a processor to open during detected patient expiration, such as by an analysis of a flow signal, but close during inspiration. Similarly, a conduit to the collector may be controlled to regulate flow to the collector by a servo mechanical valve. Optionally, the opening of the collector or flow to the collector or sensor may be controlled by a timed operation of the processor such that it will be controlled to open at a certain time during treatment and close at a certain time during treatment. For example, it may be controlled so as to collect a sample for a specified period of time (e.g., a number of breaths, a number of hours or a number of treatment sessions, etc.). In some cases, the opening and closing of the sample collector or activation of sensors may be triggered by detected conditions such as a particular sleep state. For example, the access port may be controlled to be open during a sleep state but closed if a patient is in an awake stage. A determination of sleep state may be made by any suitable process but may in some embodiments be made in accordance with the sleep condition detection technologies described in PCT Patent Application No. PCT/AU2010/000894, filed on July 14, 2010, the disclosure of which is incorporated herein by reference.

In some embodiments, the variable aperture to the collector may be controlled to have a certain collection behavior (e.g., collection at certain times or events or for a certain duration) and a sensor configured to sense a chemical of that collector may be controlled by a processor to activate upon completion of the collection period.

In another embodiment of the technology, the patient may subscribe to a service for analyzing the sample collectors or collection canisters and absorbent material. In such a case, the sample collector may be removed from the patient interface after use and transported for analysis.

In some embodiments in which a physiological sensor, including a physiological breath sensor is attached directly to a gas delivery tube or conduit, the sensor may be adapted to allow air to flow freely through the sensor, in order to insure that airflow is not obstructed. For example, the sensor may be inserted within a conduit for a patient interface. In one example, the sensor itself is constructed to permit inspiratory and expiratory gases to flow freely through the sensor. In other examples, the sensor or sensing module is configured to allow respiratory gases to flow through, around or along the sensors. In some embodiments, the sensors may be configured only to have access to expiratory gases, such as by being inserted in an expiratory conduit. In some such cases, the sensors or sample collector may be installed within a flow generator portion of the respiratory treatment apparatus rather than the patient interface and the expiratory flow may be directed to the sensor or sample collector through the expiratory conduit from the patient interface.

In some embodiments of the technology, some or all of the physiological sensor(s) may be designed or integrated with suitable structures to ensure proper orientation for measurement. For example, in the case of a respiratory treatment device, the sensors may be embedded, adhered or otherwise integrated with a cushion surface or frame of a patient interface such as a mask (e.g., a nasal mask, nose and mouth mask, or full face mask), nasal prongs or nasal pillows etc., for a respiratory treatment apparatus (e.g., a CPAP apparatus or ventilator apparatus). For breath sensing, the sensors may be oriented on an internal side of the cushion or frame proximate to a patient's mouth so as to be exposed to patient exhalation gases. In some cases, the sensing module may be positioned directly opposite the patient's nares and/or mouth within the patient interface. Alternatively, the sensors may obtain access to such gases via a conduit or port of the mask or frame when the sensors are oriented within or on an external side of the cushion or frame.

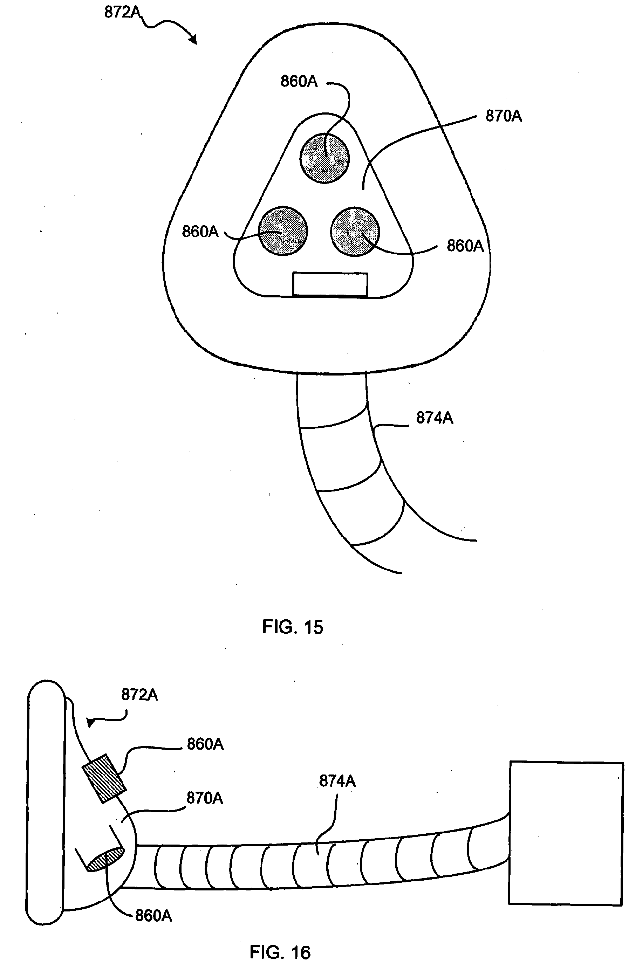

Similarly, the physiological sensor(s) may alternatively or in addition thereto, be integrated with a headgear support, such as a strap of a headgear support for the sensors or a strap of a headgear support for a patient interface of a respiratory treatment apparatus (e.g., a nasal mask, nasal prongs or full face mask). In the case of such a sensor for detecting breath, a conduit from the mask or prongs may direct a portion of exhalation gases to the sensor from the mask or prongs. Preferably, such integrated sensors may be non contact or dry contact electrochemical sensors. As described further herein, the sensors may also be electrochemical sensors adapted for analyzing saliva and mucus. In a preferred embodiment, the present technology employs the use of at least one breath sensor to sense or detect analytes in the breath of a patient. However any of the previously described sensors may be implemented either independently or in combination with multiple sensors. Examples of the integrated sensor embodiments are illustrated in FIGS. 5 through 20.

In FIG. 5, a nasal mask 500 includes a cushion 502 that when worn may be compressed to the face around a patient's nose for providing a pressure seal for a respiratory pressure treatment such as a CPAP treatment for sleep disordered breathing. Thus, one or more physiological sensor(s) 512 may be integrated with the cushion 502 or mask frame such that when the cushion is worn, the sensor(s) has a desired orientation for direct or indirect skin contact and may optionally form part of the pressure seal of the mask. In the case of a noncontact sensor, the electrodes may be embedded in the material (e.g., silicone or non-conductive polymer), of the cushion such that a non-conductive material portion of the mask cushion that is part of the pressure seal during use of the mask, lies between the skin of the patient and the embedded electrode. Optionally, the wire leads for the electrodes may be routed within or upon a portion of the patient interface or mask so that the leads may extend to electronically couple with a signal interface of a processor described herein, such as a processor of a respiratory treatment device. For example, these leads may also be integrated with a breathable gas or airflow conduit that extends from the patient interface to direct airflow from a flow generator of a respiratory treatment device. For example in a heated air delivery conduit as described in the Assignees pending U.S. Patent Application No. 12/847,021 filed July 30, 2010, the disclosure of which is incorporated herein by reference.

FIG. 6 illustrates a nose and mouth respiratory mask 600 to seal airflow or pressure at the nares and mouth of a patient from respiratory treatment apparatus similar to the mask of FIG. 5. In this example, the one or more physiological sensor(s) 612, are integrated with the mask cushion 602 or mask frame. Similar to the embodiment of FIG. 6, this mask may optionally be equipped with any selection of one or more of the physiological sensor(s) or a sample collector, such as a breath moisture or sweat collection canister described herein, or known in the art. In one embodiment of the present technology, the physiological sensor(s) 612 are non-contact electrochemical sensors, and may be used to detect physiological parameters such as pH, temperature, or other type of analytes detectable in the skin or perspiration of a patient (e.g., glucose, urea, etc.). The sample collector(s) may also be used to collect saliva from a patient.

In reference to FIGS. 6A and 6B, physiological sensor(s) 604A, 604B and 604C may be mounted to the underside of the frame 619 or 621 of the patient interface 613 or 617, respectively. Sensors of this type may be integral with the frames 619 or 621 (e.g., composite design) or be adhered using chemical adhesives or bonding agents.

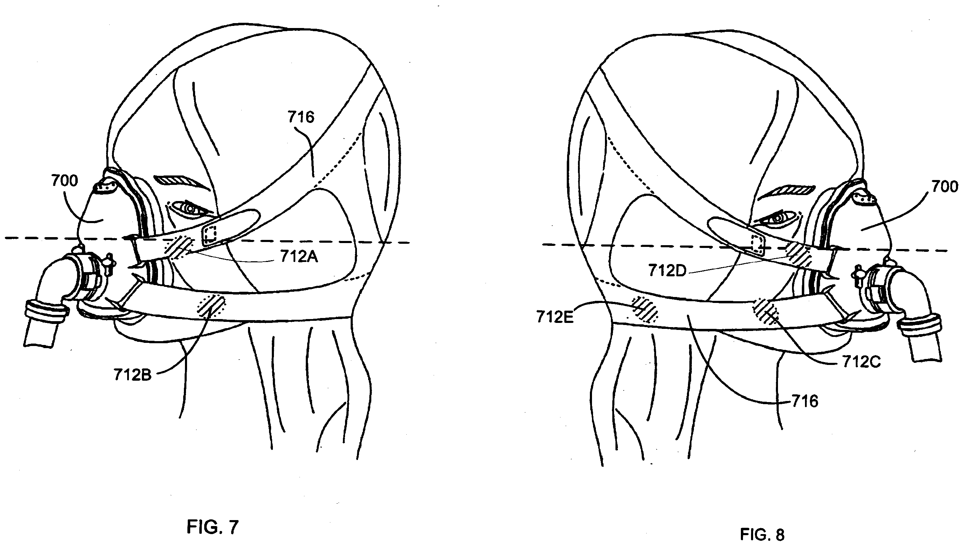

FIGS. 7 and 8 depict an example embodiment that employs one or more physiological sensor(s) integrated within the headgear support 716 for the mouth and nose mask 700, in addition to or alternatively to the sensors of the mask cushion or frame as shown on the mouth and nose mask 700. In this particular embodiment, the physiological sensor(s) 712 A, 712 B, 712 C and 712 D may be integrated with a mask strap of the headgear support 716 so that the sensors are in contact with or near the surface of the patient's skin when the mask is worn by the patient. In this example, the sensors are integrated at the underside of the straps. Optionally, a neck electrode 712 E may be integrated with the strap for skin contact at or near the neck, such as the back of the neck strap or neck strap portion of headgear support 716 as shown in FIG. 8.

In one example, the physiological sensor(s) 712 A, 712 B, 712 C, 712 D, and 712 E may be cardiac sensors or electrodes. In an alternative embodiment, the physiological sensor(s) 712 A, 712 B, 712 C, 712 D, and 712 E may be temperature sensors. In yet a further embodiment, the physiological sensor(s) 712 A, 712 B, 712 C, 712 D, and 712 E may be sensors which detect conditions in the body such as blood oxygen levels or chemicals from the skin or perspiration of a patient. In another example, the physiological sensors may be configured to detect various analytes in saliva and mucus. Optionally, the embodiment may employ any combination of these physiological sensors.

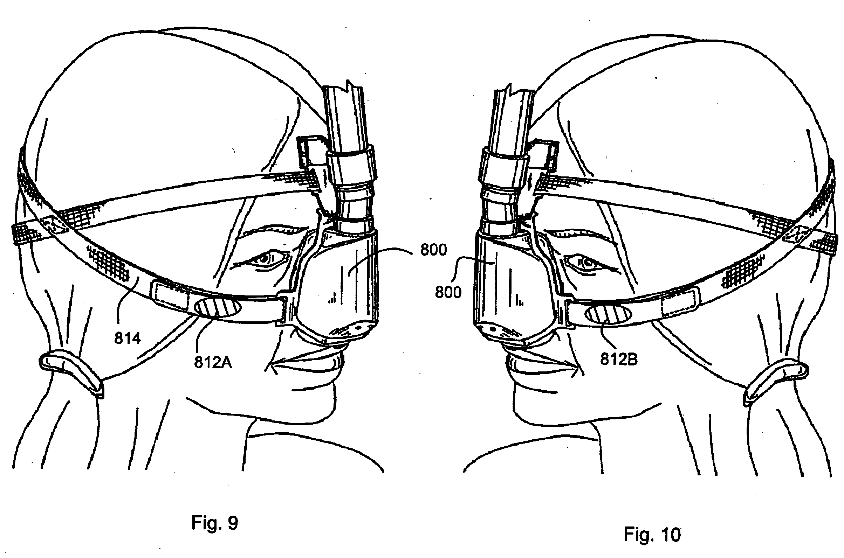

Similarly, FIGS. 9 and 10 depict a nasal mask similar to the mask 500 of FIG. 5. This example embodiment employs one or more physiological sensor(s) 812A 812B, integrated with the headgear support 814 for the nose mask 800, which may be in addition to or alternatively to the sensors of the mask cushion or frame as shown on nose and mouth mask 700. As illustrated, the physiological sensor(s) 812A 812B may be integrated with a mask strap of the headgear support 814, so that the sensors are in contact with or near the surface of the patient's skin when the mask is worn by the patient. In this example, the sensors are also integrated at the underside of the straps.

In such an embodiment any necessary or desired additional electrode(s) may also be integrated on a headgear support such as described in the FIGS. 7 10, or it may simply be in skin contact with the patient, such as being temporarily adhered to a neck of the patient.

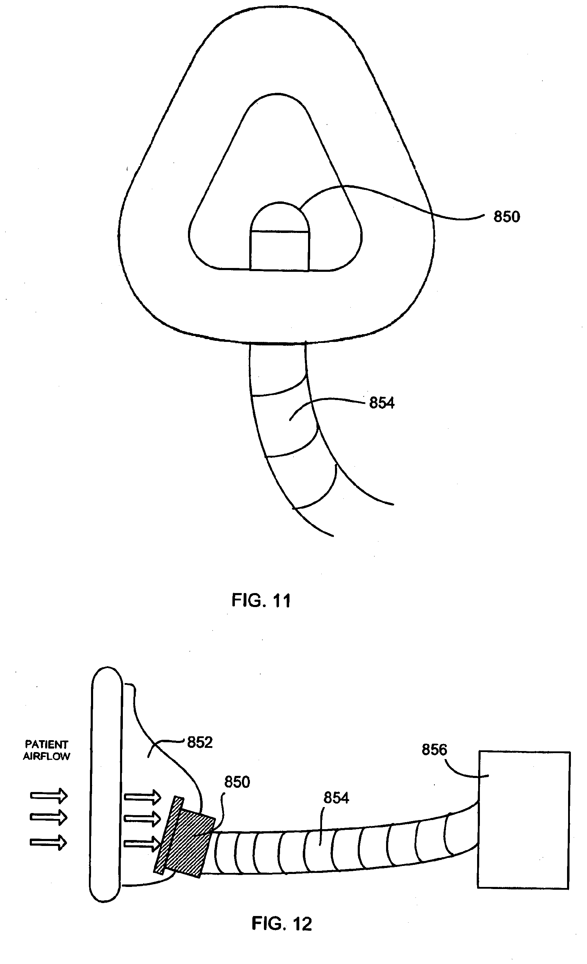

FIGS. 11 and 12 depict an example of the technology, which employs the use of a physiological breath sensor 850, to detect analytes, chemical markers and biological markers in a patient's breath (e.g., Nitrous Oxide). In the example depicted in FIG. 11, the physiological breath sensor 850 (or collector) is mounted within the patient interface 852. The physiological breath sensor 850 may be positioned proximate to the mouth to directly receive the patient's exhaled breath from within the patient interface 852. In this embodiment, the physiological breath sensor 850 is mounted on one side of the gas delivery tube or conduit 854, which attaches to respiratory treatment apparatus 856.

The physiological breath sensor 850 may also be adapted to assess samples of saliva from the patient interface. These saliva samples may be analyzed for the presence or absence of volatile and nonvolatile analytes which may include but are not limited to NH4⁺, acetate, K⁺ and Ca₂⁺.

In some cases, a wicking material could be configured on the inner surface of at least a portion of a patient interface 852. The wicking material could be a fabric or stable medical grade fiber or paper, which may be used to line all, or a portion of the patient interface 852. The fabric, fiber or paper could be used to absorb saliva samples from a patient wearing the patient interface 852. While described herein with respect to patient interface 852, the present saliva analysis technology could be used in any of the patient interfaces, or physiological sensors described herein.

Optionally, in some examples of the present technology, a saliva collector may be implemented as a part of a mandibular advancement device, such as a device worn in the mouth for adjusting the position of the jaw and tongue so as to ease breathing and prevent snoring. Such a device may optionally include a wicking material and which may optionally draw saliva into a chamber of the mandibular advancement device so that the saliva may then be analyzed.

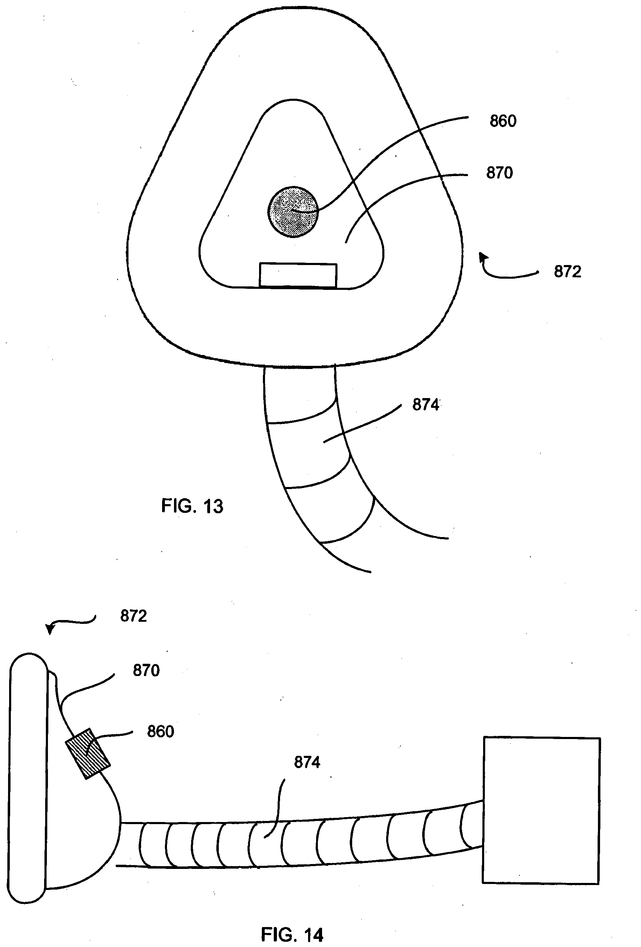

FIGS. 13-16 depict further examples of the present technology. In these particular embodiments, one or more physiological breath sensors 860 and 860A are contained in the frame 870 and 870A of a respiratory patient interface. Unlike the embodiments depicted in FIGS. 11 and 12, the physiological breath sensors 860 and 860A in this example are contained within the frame 870 and 870A of a patient interface 872 and 872A, without being connected to a gas delivery tube or conduit 874A. The physiological breath sensors 860 and 860A depicted in this example may be configured to attach to the frame 870 and 870A by the means described below. In some such cases, the sample collector or sensor may be mounted at an exhaust vent, such as by overlapping the exhaust vent or a portion thereof. In some cases, the controlled opening of a collector or sensor as previously discussed may allow exhaled air to be vented through the sensor or collector out of the mask and may thereby increase the exhaust vent flow of the mask. In some such cases, the opening of such a collector may be coordinated with a closing of a variable area exhaust vent area so as to permit maintaining of a desired level of exhaust flow from the mask or patient interface. Example variable area exhaust vents and conduits are described in U.S. Provisional Patent Application No. 61/534,044 filed on September 13, 2011, the disclosure of which is incorporated herein by reference. In some embodiments, these vents and/or conduits may be implemented as a variable aperture to regulate flow of expired gas and/or condensate to a sample collector and/or sensor.

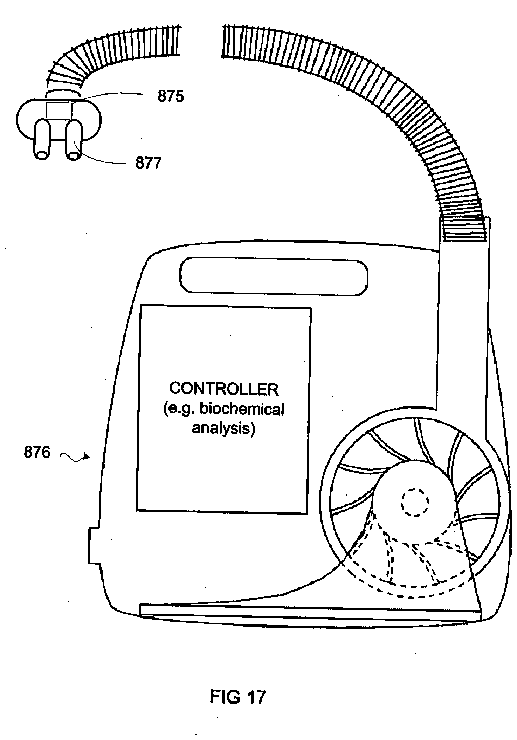

In another embodiment, depicted in FIG. 17, a physiological sensor 875 is coupled to a nasal cannula 877, as part of respiratory support system 876. In such an embodiment, the prongs of the nasal cannula may be configured as collector and/or sensors to detect various analytes in mucus and/or have a structure and/or material for touching mucosa and/or collecting mucus during use. For example, the nasal cannula 877 may be composed at least in part from silicone, which may include a stabilizing material. A stabilizing substance may be incorporated into portions of the cannula, in order to touch mucosa and/or retain mucus samples for later analysis. In such examples, the number of hours of patient use of a cannula, or use of any other sample collector or sample collecting patient interface, may be recorded. For example, a respiratory treatment apparatus may monitor the time of use and may even generate a warning or message for the user when the suitable sampling/use time has been reached. Rather than discarding or washing the cannula or sample collector at the particular time notification, it may be analyzed so as to obtain mucus samples from the nasal prongs or other collector. In such a configuration, the nasal prongs, for example, may be configured with a hydrophilic material, to allow aqueous samples to be absorbed for analysis. It may also be configured with a hydrophobic or lipid soluble material, to allow for fats to be absorbed for analysis.

Alternatively, a wicking material could be configured on the cannula. The wicking material could be a fabric or stable medical grade fiber or paper, which may be used to line all, or a portion of the nasal prongs. The fabric, fiber or paper could be used to collect mucus samples from a patient wearing the cannula.

With respect to the embodiments referenced above, and in particular those depicted in FIGS. 11 17, one or more physiological breath sensors may be attached to the frame of a patient interface, a sensing module as described above, and shown in FIGS. 4 4D. In this example of the technology, the sensing module would be securely attached to the respiratory support interface. The housing may contain the structure and functionality, which allow various sensors to be interchanged, based on patient need, and/or samples to be collected.

Examples of the present technology may also be highly useful in treating patients who are on a ventilator. Ventilator dependent patients, particularly those who require treatment for extended periods of time, often have other medical conditions (e.g., diabetes, COPD, congestive heart failure, etc.) which may pose life threatening risks. Artificial respiration with a ventilator may require endotracheal tubes, nasal intubation tubes, and tracheotomy tubes (or stomas).

In the representative examples set forth herein, the present technology may be adapted for use with these different types of ventilators or apparatuses which provide mechanical respiration.

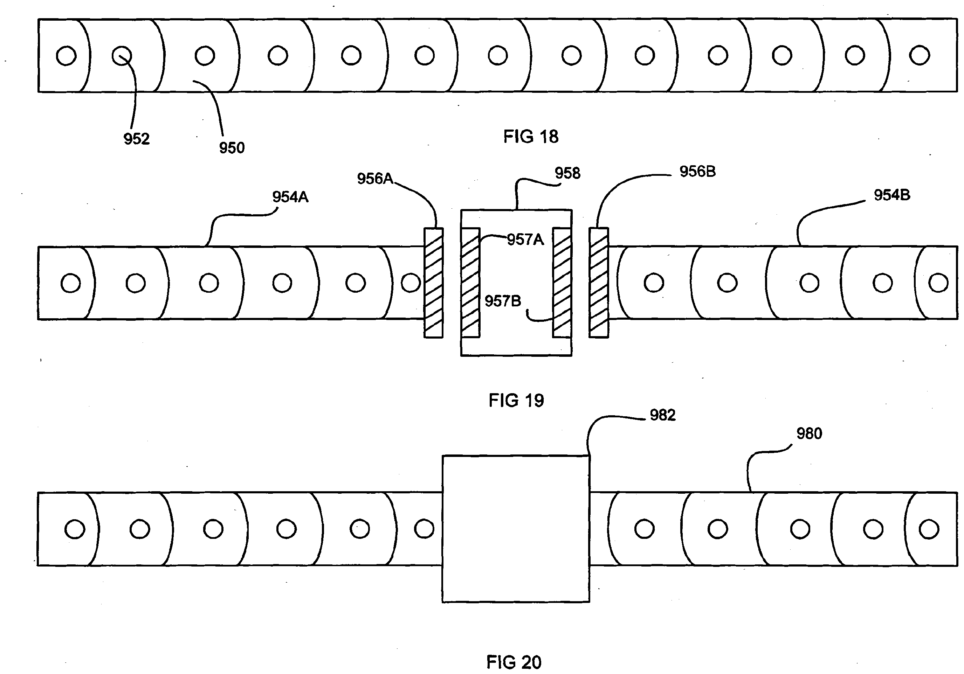

FIGS. 18 20 depict various examples of physiological sensor(s) or sample collectors integrated with a gas delivery tube or conduit, such as on that serves as an expiratory conduit. FIG. 18 is a representation of a gas delivery tube or conduit 950. In this embodiment, one or more physiological sensor(s) 952 are located on the inside of the conduit itself. In yet another embodiment of the present technology, FIG. 19 depicts a gas delivery tube or conduit 954A and 954B. Tubes 954A and 954B contain coupling mechanisms 956A and 956B, respectively at one end of each tube. These coupling mechanisms 956A and 956B allow the tubes 954A and 954B to attach to a physiological sensor or sensing module 958. In a further embodiment, the physiological sensor or sensing module may contain complementary coupling mechanisms 957A and 957B for attaching to coupling mechanisms 956A and 956B.

In yet another embodiment of the present technology, FIG. 20 depicts a gas delivery tube or conduit 980, with an integrated physiological sensor 982.

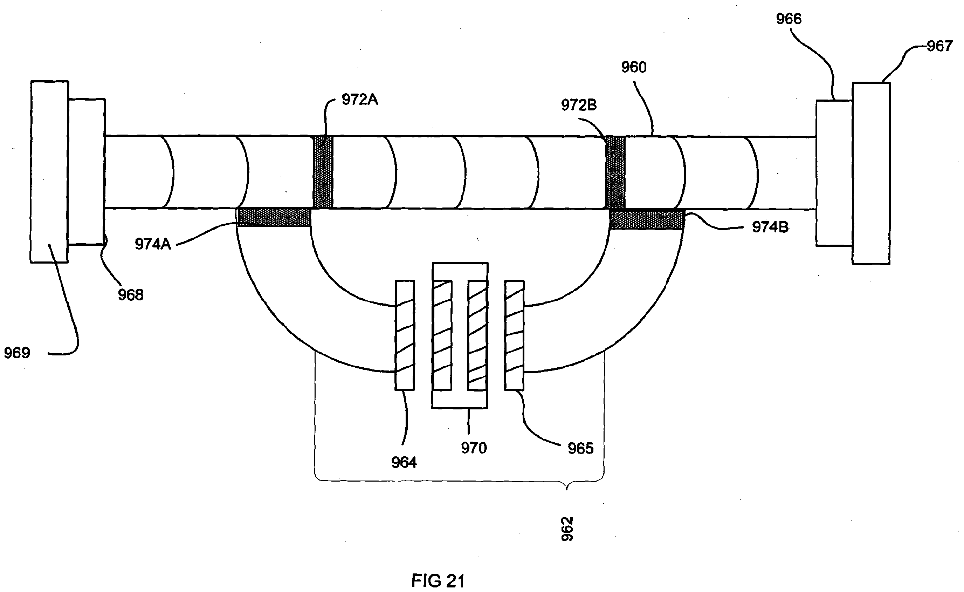

The present technology also provides a solution for accessing and changing physiological sensor(s) or sample collectors contained within a gas delivery tube or conduit, without needing to interrupt the flow of air between the respiratory treatment apparatus and the patient. FIG. 21 depicts a gas delivery tube or conduit 960, with a bypass tube 962. The gas delivery tube or conduit 960 may be attached at one end, to patient interface 969, by coupling mechanism 968. At the other end, gas delivery tube or conduit 960 may be attached to a respiratory treatment apparatus 966, by coupling mechanism 967. In this embodiment, the bypass tube 962 is configured with two coupling mechanisms, 964 and 965. The coupling mechanisms 964 and 965 attach to a physiological sensor 970, or sensor housing .

During normal operation, closure valves 972A and 972B may be closed, while bypass closure valves 974A and 974B remain open. This arrangement of valves ensure that air exchange between the patient and the respiratory treatment apparatus is only conducted through bypass tube 962, thus allowing for the physiological sensor 970 to detect analytes from the patient, and in particular, the patients breath. When access to the physiological sensor 970 is necessary, closure valves 972A and 972B are opened, and bypass closure valves 974A and 974B are closed. This valve configuration allows access to the physiological sensor 970, while maintaining the patency of airflow between the patient and the respiratory treatment apparatus.

In one form of the present technology, a cartridge is provided to a patient's mask, preferably the same mask that is used for nasal CPAP treatment of obstructive sleep apnea. The cartridge includes an adsorbent material, for example, CARBOXEN (SIGMA ALDRICH). In use, the adsorbent material is exposed to volatile compounds in exhaled breath. After exposure, the cartridge may be removed, and coupled to an analysis system. One form of cartridge comprises a sealing system so that prior to use, and upon removal of the cartridge, contaminants are not introduced. One form of cartridge contains thermal isolation and or a cooling system to prevent or reduce loss of compounds from the adsorbent material prior to analysis. In one form, the cartridge comprises a moisture absorbing material, preferably the moisture absorbing material is removable. In use, the cartridge is placed in analysis system such as a gas chromatograph to detect the presence of the relevant compound. In this way, a patient that is being treated for a sleep disorder may also be monitored for the present of other conditions, such as cancer or diabetes. For example, the patient being treated for sleep apnea may also be monitored for lung cancer.

### ELECTRICAL CONDUIT

As discussed in detail, the present technology employs the use of different types of physiological sensor(s) and in preferred embodiments, electrochemical sensors. More specifically, those electrochemical sensors may be used to detect chemical analytes in the breath of a patient. As with any electronic device, information gathered and transmitted as an electronic signal requires an electronic conduit or pathway. Accordingly, the present technology may be configured in various ways to transmit the electronic signal from the physiological sensor(s), to the respiratory treatment apparatus.

The physiological sensor(s) utilized in the present technology may have outgoing electronic circuitry to transmit signals or data regarding physiological information collection. The outgoing circuitry may be configured with the gas delivery tube or conduit. A configuration of outgoing electronic circuitry that relies on wires for data transmission is referred to herein as a "wired solution."

However, in some embodiments, the sensors themselves may be implemented with components for transmitting the physiological signals to the controller or physiological signal detection processor by wireless communication. For example, the signals interface of the physiologic signal detection processor or controller may include a receiver or transceiver to communicate wirelessly with one or more transmitters or transceivers integrated with the physiological sensor(s). In such a case, data representing the physiological signal(s) may be transmitted digitally, for example, by any suitable wireless protocol, such as Bluetooth. Optionally, a set, or array of sensors may share a common transmitter or transceiver for transmission of the data of several sensors to the controller. This approach to data transmission in the present technology is referred to herein as a "wireless solution."

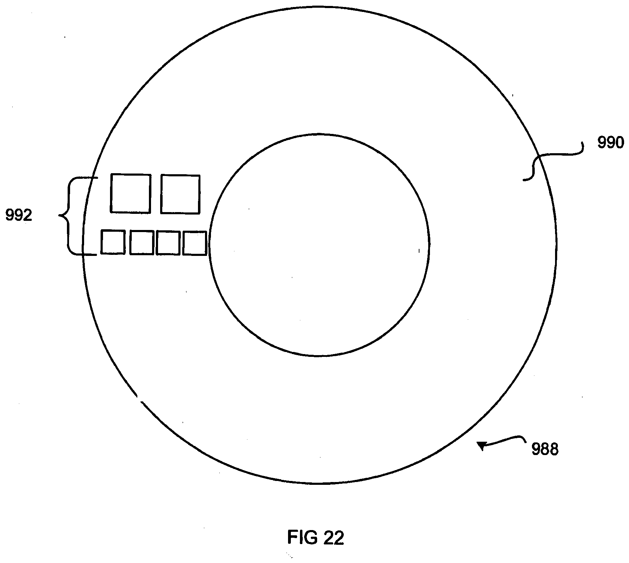

As depicted in FIG. 22, in certain examples of the present technology, the coupling mechanism 990 of physiological sensor, or sensing module 988 may be equipped with electronic contacts. The electrical contacts mate with electronic contacts on a compatible coupling mechanism (not shown) located on another section of the respiratory treatment apparatus, such as a gas delivery tube or conduit, a patient interface, and/or the flow generator module itself. In such embodiments, the wires connected to the electronic contacts transmit electrical signals via the wires, to and/or from the respiratory treatment apparatus. When a wired solution is utilized in the present technology, the wires may be integrated or embedded within the gas delivery tube or conduit. This arrangement further allows the present technology to function as an interchangeable system, as different gas delivery tubes or conduits may be interchangeable with various respiratory treatment apparatuses, various physiological sensor(s) or sensor housings, and various patient interfaces. As an alternative to embedding the wires and circuitry within the gas delivery tube or conduit, the wires and circuitry may be attached to the outside of the gas delivery tube or conduit, and enclosed by a plastic sheath, cover or envelope to protect the circuitry and wires from being damaged.

### RESPIRATORY TREATMENT APPARATUS

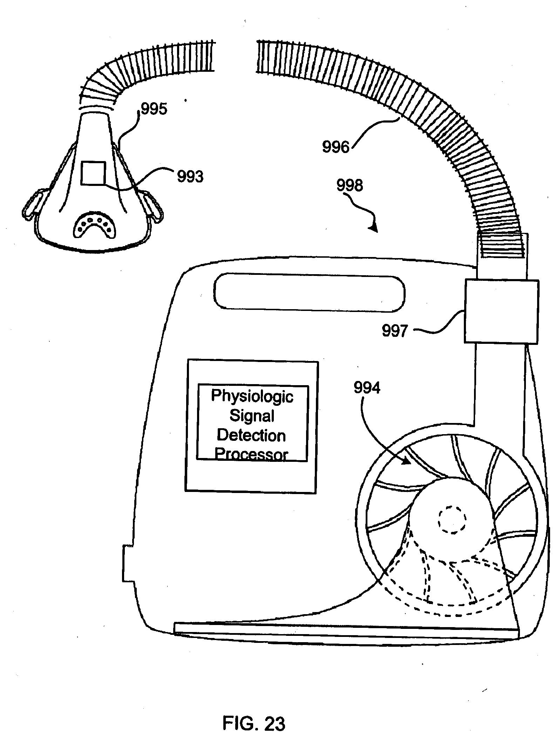

The respiratory treatment apparatus as depicted in. FIG. 23 may also be configured to provide a respiratory pressure treatment from a flow generator such as a servo-controlled blower 994. In such a case, the apparatus may optionally include a pressure sensor 993, such as a pressure transducer to measure the pressure generated by the blower 994 and generate a pressure signal p(t) indicative of the measurements of pressure. In the illustrated embodiment the pressure sensor 993 is shown located in the patient interface 995, however alternatively the pressure sensor 993 may be located in the device downstream of the blower 994. In such an arrangement a pressure compensation may be made to the pressure measured by the pressure sensor to determine the pressure at the patient interface. The pressure compensation can adjust for the pressure drop along the delivery tube 996. The flow sensor 997 may be coupled with the patient interface. The flow sensor generates a signal representative of the patient's respiratory flow. For example, flow proximate to the patients interface 106 may be measured using a pneumotachograph and differential pressure transducer or similar device such as one employing a bundle of tubes or ducts to derive a flow signal f(t). Alternatively, a pressure sensor may be implemented as a flow sensor and a flow signal may be generated based on the changes in pressure. Although the flow sensor 997 is illustrated in a housing of the treatment apparatus 998, it may optionally be located closer to the patient, such as in the patient interface 995 or delivery tube 996. Other apparatuses for generating a respiratory flow signal may also be implemented.

Signals from the sensors of the present technology may be sent to a controller or physiological signal detection processor. Optional analog to digital (A/D) converters/samplers (not shown separately) may be utilized in the event that supplied signals from the sensors are not in digital form and the controller is a digital controller.

The controller may optionally include a display such as one or more warning lights (e.g., one or more light emitting diodes). The display device may also be implemented as a display screen such as an LCD. Optionally, the display device may be controlled to show data derived from the physiological signals.

Based on flow f(t), pressure p(t) and detected physiological signals, the controller 1000 with one or more processors can generate blower control signals. For example, the controller may generate a desired pressure set point and servo control the blower to meet the set point by comparing the setpoint with the measured condition of the pressure sensor. Thus, the controller 1000 may make controlled changes to the pressure delivered to the patient interface by the blower, entirely, or in part, based on the detected physiological signals. Optionally, such changes to pressure may be implemented by controlling an exhaust with a mechanical release valve to increase or decrease the exhaust while maintaining a relatively constant blower speed. With such a controller or processor, the apparatus can be used for many different pressure treatment therapies, such as the pressure treatments for sleep disordered breathing, Cheyne Stokes Respiration, respiratory acidosis, respiratory alkalosis, asthma, COPD, diabetic ketoacidosis, or obstructive sleep apnea (e.g., CPAP, APAP, Bi Level CPAP, Auto VPAP, etc.) by adjusting a suitable pressure delivery equation. Optionally, the controller 1204 may also be implemented to make changes to pressure treatment based on detected changes of the metrics derived or detected from the sensors and collectors described herein.

### EXAMPLE CONTROLLER ARCHITECTURE

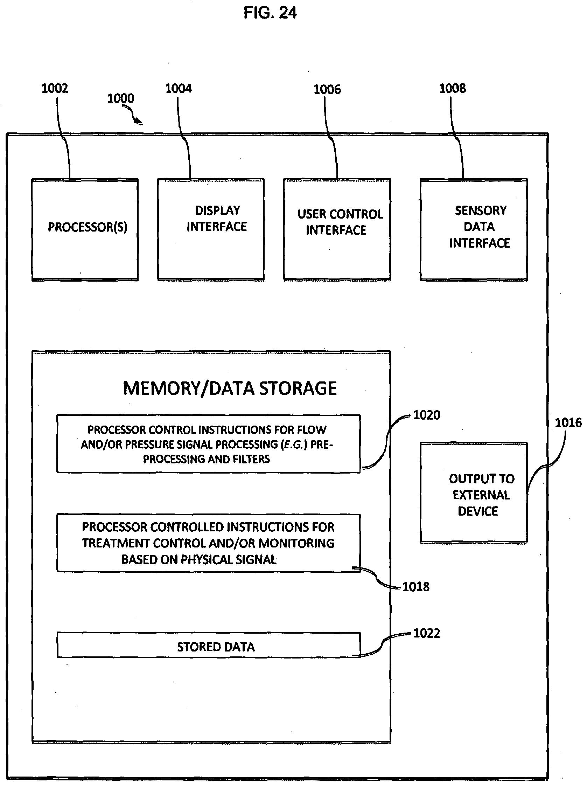

An example system architecture of a controller suitable for the present technology is illustrated in the block diagram of FIG. 24. In the illustration, the controller 1000 may include one or more processors 1002 for physiological signals as well as the respiratory treatment apparatus. Alternatively, the controller 100 may include more than one processor, each to be used for processing different types of data, or to increase the computing power of the overall controller. The system may also include a display interface 1004 to output event detection reports (e.g., respiratory rate, heart rate variability, analyte profiles, etc.), results or graphs such as on a monitor or LCD panel. A user control/input interface 1006, for example, for a keyboard, touch panel, control buttons, mouse etc. may also be provided to activate or modify the control methodologies described herein. The system may also include a sensor or data interface 1008, such as a bus, for receiving/transmitting data such as programming instructions, pressure and flow signals, facial physiological signals, breath collection related signals, breath chemical signals, cardiac signals etc. The device may also typically include memory/data storage components 1020 containing control instructions of the methodologies discussed herein. These may include processor control instructions for flow and/or pressure signal processing (e.g., pre-processing methods, filters) at 1020. These may also include processor control instructions 1018 for treatment control and/or monitoring based on physiological signal detection (e.g., nitrous oxide, CO2, acetone, pH, pathogens, etc.). Finally, they may also include stored data 1022 for these methodologies such as physiological signals, historic lookup data, critical thresholds, zone maps to determine "danger zones," etc.)

In some embodiments, these processor control instructions and data for controlling the above described methodologies may be contained in a computer readable recording medium as software for use by a general purpose computer so that the general purpose computer may serve as a specific purpose computer according to any of the methodologies discussed herein upon loading the software into the general purpose computer.

While the physiological signal detection technology has been described in several embodiments, it is to be understood that these embodiments are merely illustrative of the technology. Further modifications may be devised within the spirit and scope of this description. For example, while an integrated apparatus is contemplated by the present technology, the methodology of the components of the apparatuses described herein may be shared across multiple components of a system. For example, a controller may simply measure the physiological signals of the patient and transfer the data representing those signals to another processing system. The second processing system may in turn analyze the data to determine the physiological signal or related data and metrics therefrom. The second processing system may then evaluate the data and generate warning messages as described herein, such as by sending one or more of the described messages, in electronic form for example, back to the patient monitoring device for display on the device to warn the patient. Other variations can be made without departing with the spirit and scope of the technology.

### EXAMPLE METHODOLOGY

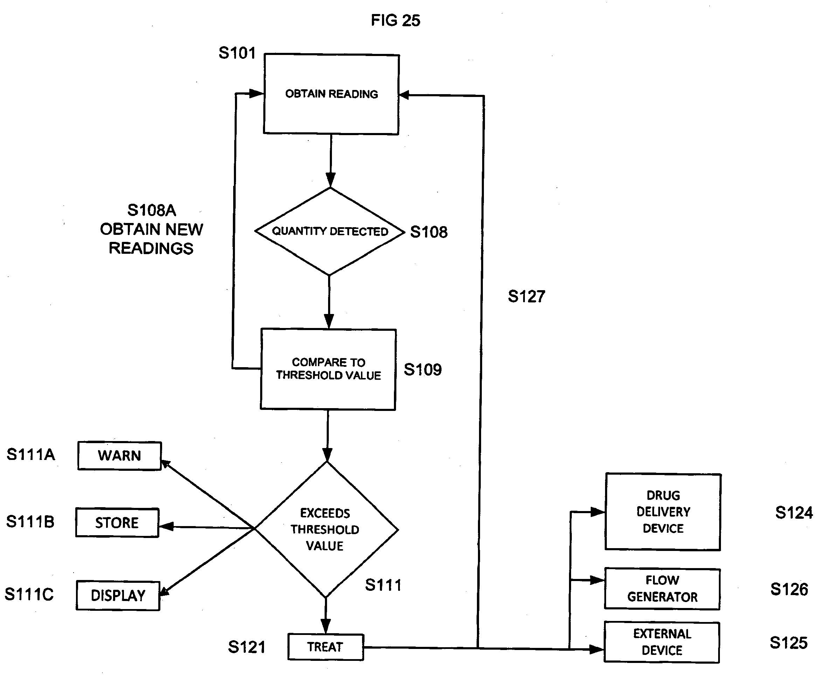

FIG. 25 is a flow diagram illustrating a logic sequence executed by a controller integrated with, or attached to a respiratory treatment apparatus. At S101, a physiological sensor(s) obtains a reading. In the case of some electrochemical sensors, a chemical reaction may take place on the sensor, which may indicate the presence or absence of at least one analyte. The sensor then converts this signal into an electronic signal, representative of the information obtained from the chemical reaction. The sensors may be programmed to take readings at variable intervals and durations, depending on clinical need or based on a triggering event as previously discussed. After a reading is obtained, the signal is transmitted by wired or wirelessly (as described above), to the controller. The process proceeds to S108, in which the presence or absence, or quantity, of an analyte is detected. If no analyte is detected, the processor may direct the sensor to continue obtaining readings at S108A or delay for a next sensing cycle to allow further sample accumulation by the collector.

At S109, the processor compares the analyte value to a one or more threshold values. Based on the threshold comparison (s), at step S111, one or more of processes may be triggered. The triggered processes may include any combination of the following: a warning event S111A, in which an audible, and/or visual alarm is triggered; a storing or recording event S111B, in which data concerning the detected analyte is stored or recorded; a display event S111C, in which the triggering event is displayed on the display of the controller, or an external device, such as a remote computer monitor in a telemetry station; and/or a treating event S121 (described below).

Optionally, the threshold may be an empirically determined threshold or a threshold derived from a prior measurement by the sensor of the patient's breath. In such a case, the threshold comparison may be an indication of a recent change or a significant change (depending on the threshold) in the level of the detected analyte. Based on such a comparison,

Optionally, the processor may initiate or adjust a treatment protocol S121 based on the detection of the analyte. For example, the processor may control the flow generator at S123, to adjust its treatment protocol (e.g., pressure, flow, timing based on information yielded from comparison of the analyte value. For example, based on the comparison, the controller may initiate delivery of a pressure support treatment (e.g., a Bi level therapy) from a CPAP treatment.

Optionally, at S124 the processor may also control activation of a drug delivery device, to deliver medication to a patient based on signal parameters. In addition, the processor may send a signal via wired, or wireless communication, to an external device in step S125, based on the detection of the sensors. For example, indication of a diabetic event may be communicated to a patient's implanted insulin pump to increase or decrease insulin accordingly. Also, the processor may control the flow generator based on the detected physiological data. In yet another option, the processor may activate additional sensors of the sensing module or control more frequent, and/or longer sensing by the sensors at S127.

### DIABETES EXAMPLE

The present technology may be particularly useful in treating patients with co morbidities such as diabetes and respiratory related diseases. Diabetes causes. multiple treatment challenges. Patients in advanced stages of disease may present with metabolic conditions such as renal failure, neuropathy, vascular insufficiency and diabetic ketoacidosis (DKA), and such presentation may signal the onset or sustenance of an emergent diabetic crisis. The present technology may be adapted to address and treat such conditions by providing co treatment solutions to the patient and caregiver.

In one example, the present technology is adapted with electrochemical sensors for detecting acetone in the breath of a patient attached to a respiratory support system. In such an instance, a respiratory treatment apparatus may be adapted with a patient interface coupled with any configuration of sensors as previously described. For example, in the case of a respiratory treatment apparatus suitable for a diabetic patient, the apparatus may be equipped with one or more electrochemical sensors for detecting acetone in the breath of a patient.

The pathophysiological factors involved in DKA involve, in part, the breakdown of ketones. Diabetic patients, and in particular, patients with Type I diabetes have poor, or no insulin regulation of blood glucose levels. While these patients may have sufficient, or even excess levels of glucose, insulin insufficiency prevents the body from processing the glucose for energy. In the absence of proper glucose metabolism, the body digests protein and fat as fuel. This type of metabolism involves the breakdown of fatty acids into ketone bodies. In diabetic patients, the accumulation of keto acids, such as acetoacetic acid may lead to a drastically reduced blood pH, which in some cases can be fatal.

One of the metabolic by products of acetoacetic acid is acetone. This acetone can often be detected in the breath, and presents as a sweet or fruity smelling substance on the breath of a patient, and is often mistaken for alcohol.

Due to the accumulation in the blood of acidic ketone bodies, the CO₂ level in the blood also increases as the acid is broken down. The excess blood CO₂ levels may cause a patient to present with rapid, labored breathing known as tachypnea. More specifically, in the case of metabolic acidosis such as DKA, this type of breathing is referred to as Kussmaul breathing. Kussmaul breathing is one way in which the body attempts to rid itself of excess CO₂. However, this type of breathing is physiologically inefficient, and may result in hypoxia.

In an exemplary embodiment of the present technology, a respiratory treatment apparatus may be configured with physiological breath sensors to detect the presence of acetone. Optionally, the controller of the apparatus may also employ readings from pulse oximeter and a flow sensor to evaluate the presence of a diabetic condition from the paCO₂ and breathing pattern. Detection of one or more of acetone, elevated levels of paCO₂ and Kussmaul breathing by the processor may trigger one or more events programmed into the controller of the respiratory support system such as the controlled treatment responses described herein.

For example, after detection of an unacceptable level of acetone in a patient, the processor of the present technology may control the flow generator of the respiratory treatment apparatus to increase pressure support, in order to decrease the patient's carbon dioxide levels. Concurrently, or independently, the controller may control a peripheral insulin pump worn by the patient to increase insulin output. Additionally, the controller may also signal an infusion pump to adjust dosing of medication according to the algorithm outputs. For example, if a patient were receiving glucagon intravenously through an infusion pump, the processor of the respiratory support system may control the infusion pump to increase the dosing or administration of the glucagon.

### CASCADING SENSORS

As contemplated in the present technology, multiple sensors may be implemented simultaneously to detect various analytes associated with particular disease processes. While multiple sensors may actually be present in a respiratory patient interface and actively monitoring at all times, the present technology may employ the use of a system that allows less than all of the sensors to be active at all times and may permit activation of the sensors (e.g., wake from a sleep mode) in a cascade as the sensors are needed. In such a case, one or more sensors may be selectively activated by the controller in response to events detected from signals generated by one or more other sensors. For example, as previously mentioned, a sensor that analyzes a breath condensate may be activated or triggered in association with a detection of patient respiration that is made based on a respiration signal from a pressure sensor or flow sensor. In another example, one sensor may be active to detect a baseline analyte. If the analyte is sensed, such that the detection meets a certain preset level such as by a comparison of a measure based on a signal from sensor and a threshold, the system may trigger other physiological sensor(s) as part of a sensor cascade. Deployment of multiple sensors may yield better energy efficiency, in that it reduces the amount of time the sensors are operating. It may also reduce the wear on sensors that have limited use capabilities, when sensing does not need to be continuous. Such functionality may allow the present technology to operate at a maximally efficient level, until additional resources are needed from the system.

Cascading sensor arrays may be used in the technology to detect any type of analyte indicated in any related disease or metabolic condition and may be implemented for detection of many diseases or patient conditions. As previously mentioned, sensors may be available in packages or kits based on the desire to detect conditions related to one or more diseases and may be equipped for cascading operation. For example, an asthmatic patient may purchase a kit containing pH and NO sensors, while a diabetic patient may purchase a kit containing acetone and glucose sensors.

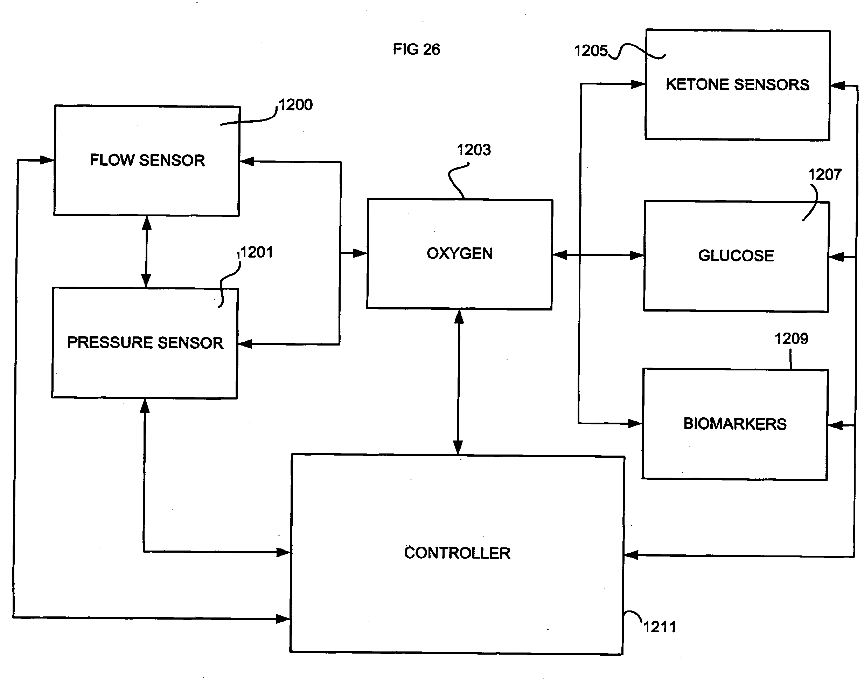

In one example, as shown in FIG. 26, a respiratory treatment apparatus may be configured with a cascading sensor configuration specifically designed for diabetes detection and treatment. In this particular example of the technology, the respiratory treatment apparatus may also include specific sensors for detecting analytes associated with diabetes and diabetic related metabolism, as described in the preceding example. This particular embodiment may contain at least one pulse oximetry sensor 1203, at least one sensor for detecting ketones 1205 such as acetone, and at least one glucose sensor 1207. The system may also include at least one sensor for detecting biomarkers 1209 (e.g., cell mediators, immune markers). For example, such sensor(s) 1209 may detect immunoglobulin E (IgE), and inflammatory cytokines such as interleukins (IL). Various interleukins such as IL 1alpha, IL 6, IL 4 and IL 10 are known to be involved in the inflammatory response and associated with diseases such as diabetes and asthma, and may be detected by sensor (s) 1209. In some such cases, the detection of low paO₂ or high paCO₂ may awaken the previously inactive acetone sensor and/or glucose sensor for an evaluation of the patient's condition by these sensors.

In one further form of the present technology, physical detection sensors such as pulse oximeters, ECG and/or EMG is combined with physiological detectors such as chemical sensors, biochemical sensors and sample collectors. For example, the oxygen saturation may be monitored. In one form the physical detection sensor is attached to, or formed as part of the patient interface, e.g. a nasal mask. The sensor is associated with a patient skin contacting portion of the mask, such as the cushion or forehead support. Furthermore, information from the physical detection sensor is combined with information from the physiological detector to reach a conclusion about the state of the patient.

In the foregoing description and in the accompanying drawings, specific terminology, equations and drawing symbols are set forth to provide a thorough understanding of the present technology. In some instances, the terminology and symbols may imply specific details that are not required to practice the technology. For example, although process steps in the assessment methodologies have been described or illustrated in the figures in an order, such an ordering is not required. Those skilled in the art will recognize that such ordering may be modified and/or aspects thereof may be conducted in parallel.

Moreover, although the technology herein has been described with reference to particular embodiments, it is to be understood that these embodiments are merely illustrative of the principles and applications of the technology. It is therefore to be understood that numerous modifications may be made to the illustrative embodiments and that other arrangements may be devised without departing from the spirit and scope of the technology.

The following aspects are preferred embodiments of the invention:
1. An apparatus for assessing a condition of a patient comprising:
   a patient interface, the interface for communicating a treatment generated by a respiratory treatment apparatus to the respiratory system of a patient; and
   a sensing module, the sensing module adapted for removable coupling with the patient interface, the sensing module including a collector to accumulate exhaled breath that is exhaled to the patient interface.
2. The apparatus of aspect 1 wherein the sensing module comprises a plurality of sample collectors.
3. The apparatus of aspect 1 wherein the collector is adapted for replacement within the sensing module.
4. The apparatus of aspect 1 wherein the collector comprises an absorbent material.
5. The apparatus of aspect 4 wherein the absorbent material includes a preservative for preserving an analyte collected from the exhaled breath.
6. The apparatus of aspect 1 wherein the sensing module further comprises an electrochemical sensor, the electrochemical sensor configured to sense a chemical in breath condensate accumulated by the collector.
7. The apparatus of aspect 6 wherein the sensing module comprises a plurality of electrochemical sensors.
8. The apparatus of aspect 1 further comprising:
   said respiratory treatment apparatus, the respiratory treatment including a flow generator and a controller, including at least one processor, to control the flow generator to generate a pressure treatment to the patient interface, and
   an electrochemical sensor, the electrochemical sensor configured to sense a chemical accumulated by the collector and generate a signal indicative of the chemical of the collector,
   wherein the controller is coupled with the sensor and the processor is configured to store a measure derived from the signal.
9. The apparatus of aspect 8 wherein the sensor is integrated with a housing of the flow generator.
10. The apparatus of aspect 8 wherein the sensing module comprises an electrochemical sensor.
11. The apparatus of aspect 8 wherein the processor is configured to synchronize an activation of the electrochemical sensor with an expiratory phase of a detected breath cycle.
12. The apparatus of aspect 8 wherein the processor is configured to activate the electrochemical sensor after a set period of time.
13. The apparatus of aspect 12 wherein the set period of time comprises a number of treatment sessions with the respiratory treatment apparatus.
14. The apparatus of aspect 12 wherein the set period of time comprises a breath cycle count.
15. The apparatus of aspect 12 wherein the set period of time comprises a timed period of use of the respiratory treatment apparatus.
16. The apparatus of aspect 8 wherein the collector of the sensing module comprises a variable aperture controlled by the processor.
17. The apparatus of aspect 16 wherein the processor is configured.to synchronize opening of the variable aperture with an expiratory phase of a detected breath cycle.
18. The apparatus of aspect 16 wherein the processor is configured to open the variable aperture for a set period of time.
19. The apparatus of aspect 18 wherein the set period of time comprises a breath cycle count.
20. The apparatus of aspect 18 wherein the set period of time comprises a timed period of use of the respiratory treatment apparatus.
21. The apparatus of aspect 18 wherein the set period of time comprises a number of treatment sessions with the respiratory treatment apparatus.
22. The apparatus of aspect 8 wherein the processor is configured to compare a measure derived from the signal and a threshold value.
23. The apparatus of aspect 22 wherein the processor is configured to generate a warning based on the comparison.
24. The apparatus of aspect 22 wherein the processor detects a quantity of an analyte from exhaled breath.
25. The apparatus of aspect 22 wherein the sensing module comprises one or more of a peroxide sensor, a nitrous oxide sensor, an acetone sensor, a carbon dioxide sensor, a pH sensor, a glucose sensor, and a lactate sensor.
26. An apparatus for assessing a condition of a patient comprising:
   a patient interface configured to direct a flow of breathable gas;
   a flow generator, adapted to couple with the patient interface,
   the flow generator configured to generate a flow of breathable gas to the patient interface,
   a controller, including at least one processor, the controller being configured to control a pressure treatment protocol with the flow generator; and
   a sensing module, including at least one electrochemical sensor,
   the sensing module adapted for coupling with the patient interface, the sensing module including a collector to accumulate exhaled breath condensate that is exhaled to the patient interface, the electrochemical sensor configured to sense a chemical accumulated by the collector and generate a signal indicative of the chemical of the collector.
27. The apparatus of aspect 26 wherein the controller is coupled with the sensor and the processor is configured to store a measure derived from the signal.
28. The apparatus of aspect 27 wherein the sensing module comprises a plurality of sample collectors.
29. The apparatus of aspect 27 wherein the collector is adapted for replacement within the sensing module.
30. The apparatus of aspect 29 wherein the collector comprises an absorbent material.
31. The apparatus of aspect 30 wherein the absorbent material includes a preservative for preserving an analyte collected from the exhaled breath.
32. The apparatus of aspect 27 wherein the sensing module comprises one or more of a peroxide sensor, a nitrous oxide sensor, an acetone sensor, a carbon dioxide sensor, a pH sensor, glucose sensor, and a lactate sensor.
33. The apparatus of aspect 32 wherein the processor is configured to synchronize an activation of one or more of the sensors of the sensing module with an expiratory phase of a detected breath cycle.
34. The apparatus of aspect 32 wherein the processor is configured to activate one or more of the sensors of the sensing module after a set period of time.
35. The apparatus of aspect 34 wherein the set period of time comprises a number of treatment sessions with the apparatus.
36. The apparatus of aspect 34 wherein the set period of time comprises a breath cycle count.
37. The apparatus of aspect 36 wherein the set period of time comprises a timed period of use of the apparatus.
38. The apparatus of aspect 32 wherein the collector of the sensing module comprises a variable aperture controlled by the processor.
39. The apparatus of aspect 38 wherein the processor is configured to synchronize opening of the variable aperture with an expiratory phase of a detected breath cycle.
40. The apparatus of aspect 38 wherein the processor is configured to open the variable aperture for a set period of time.
41. The apparatus of aspect 40 wherein the set period of time comprises a breath cycle count.
42. The apparatus of aspect 40 wherein the set period of time comprises a timed period of use of the apparatus.
43. The apparatus of aspect 42 wherein the set period of time comprises a number of treatment sessions with the apparatus.
44. The apparatus of aspect 32 wherein the processor is configured to compare a measure derived from the signal and a threshold value.
45. The apparatus of aspect 44 wherein the processor is configured to generate a warning based on the comparison.
46. The apparatus of aspect 45 wherein the processor detects a quantity of an analyte from exhaled breath.
47. The apparatus of aspect 28 further comprising a sample collector to collect saliva.
48. The apparatus of aspect 10 wherein the electrochemical sensor is configured to detect a quantity of an analyte from saliva.
49. The apparatus of aspect 10 wherein the electrochemical sensor is configured to detect a quantity of an analyte from mucus.
50. The apparatus of aspect 48 wherein the analyte is one or more of NH₄⁺, acetate, NH₃⁺, and Ca⁺.
51. An apparatus for assessing a condition of a patient comprising:
   a patient interface, the interface for communicating a treatment generated by a respiratory treatment apparatus to the respiratory system of a patient, the patient interface comprising a sensing module, the sensing module configured as a collector to accumulate mucus that is in contact with the patient interface.
52. The apparatus of aspect 51 wherein the sensing module further comprises an electrochemical sensor, the electrochemical sensor configured to sense a chemical in mucus accumulated by the collector.
53. The apparatus of aspect 51 wherein the patient interface comprises a nasal cannula and the sensing module comprises at least one prong of the cannula to contact mucosa.
54. An apparatus for assessing a condition of a patient comprising:
   a patient interface, the interface for communicating a treatment generated by a respiratory treatment apparatus to the respiratory system of a patient, the patient interface comprising a sensing module, the sensing module configured as a collector to accumulate saliva that is in contact with the patient interface.
55. The apparatus of aspect 54 wherein the sensing module further comprises an electrochemical sensor, the electrochemical sensor configured to sense a chemical in saliva accumulated by the collector.
56. An apparatus for assessing a condition of a patient comprising:
   a mandibular advancement device, the mandibular advancement device comprising a sensing material, the material configured as a collector to accumulate saliva that is in contact with the mandibular advancement device.
57. The apparatus of aspect 56 wherein the mandibular advancement device further comprising a collection chamber.

## Claims

1. An apparatus for assessing a condition of a patient comprising:
a patient interface, the interface for communicating a treatment generated by a respiratory treatment apparatus to the respiratory system of a patient; and
a sensing module, the sensing module adapted for removable coupling with the patient interface, the sensing module including a collector to accumulate exhaled breath that is exhaled to the patient interface, wherein the sensing module further comprises an electrochemical sensor, the electrochemical sensor configured to sense a chemical accumulated by the collector and to generate a signal indicative of the chemical in the collector.

2. The apparatus of claim 1, wherein the chemical is carbon dioxide.

3. The apparatus of claim 1 or 2, wherein the collector is adapted for replacement within the sensing module.

4. The apparatus of any one of the preceding claims, wherein the electrochemical sensor is configured to sense a quantity of the chemical

5. The apparatus of any one of the preceding claims, further comprising:
the respiratory treatment apparatus, including a flow generator and a controller, including at least one processor, which controls the flow generator to generate a pressure treatment to the patient interface, wherein the controller is in communication with the sensor and the at least one processor is configured to store a measure derived from the signal.

6. The apparatus of any one of claims 1 to 4, further comprising:
the respiratory treatment apparatus, including a flow generator and a controller, including at least one processor, to control the flow generator to generate a pressure treatment to the patient interface, wherein the controller is in communication with the sensor and the at least one processor is configured to adjust a treatment protocol of the flow generator in response to the signal from the sensor.

7. The apparatus of claim 5 or 6, wherein the sensor is integrated with a housing of the flow generator.

8. The apparatus of any of claims 5 to 7, wherein the at least one processor is configured to synchronize an activation of the electrochemical sensor with an expiratory phase of a detected breath cycle.

9. The apparatus of any of claims 5 to 7, wherein the processor is configured to activate the electrochemical sensor after a set period of time.

10. The apparatus of claim 9, wherein the set period of time comprises a number of treatment sessions with the respiratory treatment apparatus.

11. The apparatus of claim 9, wherein the set period of time comprises a breath cycle count.

12. The apparatus of any of claims 5 to 11, wherein the collector of the sensing module comprises a variable aperture controlled by the processor.

13. The apparatus of claim 12, wherein the processor is configured to synchronize opening of the variable aperture with an expiratory phase of a detected breath cycle.

14. The apparatus of claim 12 wherein the processor is configured to open the variable aperture for a set period of time.
